# EUROPÄISCHE PATENTSCHRIFT

(11) **EP 2 673 557 B1**
(45) Veröffentlichungstag und Bekanntmachung des Hinweises auf die Patenterteilung: **24.08.2016**
(21) Anmeldenummer: 12707743.6
(22) Anmeldetag: 02.03.2012
(51) Int. Cl.: A61B 90/30, F21V 14/02, F21V 23/04, F21W 131/205, F21Y 113/00, F21V 5/04, F21V 21/30, F21V 21/40, F21Y 115/10

(54) **OPERATIONSLEUCHTE UND VERFAHREN ZUM AUSLEUCHTEN EINER OPERATIONSSTELLE**
SURGICAL LIGHT AND METHOD FOR ILLUMINATING A SURGICAL SITE
LAMPE OPÉRATOIRE ET PROCÉDÉ POUR ÉCLAIRER UN CHAMP OPÉRATOIRE

(30) Priorität: 02.03.2011 EP 11156645
(43) Veröffentlichungstag der Anmeldung: 18.12.2013
(73) Patentinhaber: TRUMPF Medizin Systeme GmbH + Co. KG, 07318 Saalfeld (DE)
(72) Erfinder: MARKA, Rudolf, 85737 Ismaning (DE); ROSENHEIMER, Rouven, 81377 München (DE)
(74) Vertreter: Prüfer & Partner mbB Patentanwälte · Rechtsanwälte
(86) Internationale Anmeldenummer: PCT/EP2012/053682
(87) Internationale Veröffentlichungsnummer: WO 2012/117108

(56) Entgegenhaltungen:
- EP-A1- 2 136 128
- EP-A1- 2 215 987
- EP-A1- 2 283 790
- EP-A2- 0 299 196
- EP-A2- 0 422 331
- US-A- 5 068 767

## Beschreibung

Die Erfindung bezieht sich auf eine Operationsleuchte, insbesondere auf eine Operationsleuchte, die ein Leuchtfeld bereitstellt, dessen Leuchtfelddurchmesser sich nach einer Veränderung des Arbeitsabstands nicht verändert.

Um Operationen unter für den Operateur günstigen Bedingungen durchzuführen, ist unter anderem eine gute Ausleuchtung des Operationsfelds erforderlich. Dies kann üblicherweise durch Einstellen verschiedener Parameter der Operationsleuchte erreicht werden. Dazu sind in der Regel eine Position und eine Orientierung des Leuchtenkörpers, eine Fokussierung der Lichtstrahlen auf die Operationsstelle und eine Lichtstärke des abgestrahlten Lichts, also die Beleuchtungsstärke auf der Operationsstelle, einstellbar. Ein Verändern der Position und der Orientierung sowie die Leuchtfeldzusammenführung werden üblicherweise durch den Operateur selbst durchgeführt, der dazu den Leuchtenkörper an einem sterilen Handgriff nimmt, und an die gewünschte Position und in die gewünschte Orientierung schwenkt. Durch Verdrehen des sterilen Handgriffs wird dann üblicherweise die Leuchtfeldzusammenführung, d.h. der Abstand des Schnittpunkts der abgestrahlten Lichtstrahlen von dem Leuchtenkörper verstellt.

Es gibt inzwischen Operationsleuchten, bei denen der Abstand zwischen dem Leuchtenkörper und der Operationsstelle gemessen wird, und die Lichtstärke des abgestrahlten Lichts entsprechend einer Arbeitsabstandsänderung korrigiert wird, so dass die zentrale Beleuchtungsstärke auf der Operationsstelle unverändert bleibt. Dabei wird aber nur die Beleuchtungsstärke, aber nicht der Leuchtfelddurchmesser angepasst, da die Lichtquellen in diesem System starr angeordnet sind, und Parallelstrahler, die dieses Problem teilweise lösen würden, mit diesem Optikkonzept nicht realisierbar sind.

Daraus ergibt sich die Aufgabe, eine Operationsleuchte bereitzustellen, die bei einer Veränderung des Arbeitsabstands den Leuchtfelddurchmesser unverändert beibehält.

Die Aufgabe wird durch einen Gegenstand mit den Merkmalen des Anspruchs 1 oder des Anspruchs 21 und ein Verfahren mit den Merkmalen des Anspruchs 16 oder des Anspruchs 34 gelöst. Weiterentwicklungen der Erfindung sind Gegenstand der abhängigen Ansprüche.

Eine Operationsleuchte zum Ausleuchten einer in einem gewählten Abstand angeordneten Operationsstelle ist dazu so ausgebildet, dass sie gemäß einem Aspekt zumindest einen Leuchtenkörper aufweist, der mindestens eine erste Lichtquelle und eine zweite Lichtquelle aufweist, wobei die Lichtquellen angepasst sind, jeweils ein erstes Leuchtfeld und ein zweites Leuchtfeld mit unterschiedlichen Durchmessern zu bilden, die ein resultierendes im Wesentlichen kreisförmiges Leuchtfeld mit einer Operationsleuchtennorm-gerechten Lichtverteilung mit einer voreingestellten normgerechten relativen Beleuchtungsstärke bei einem vorbestimmten Durchmesser ergeben, und eine Steuerungsvorrichtung für die Lichtquellen aufweist, die dazu angepasst ist, die Lichtstärke der ersten Lichtquelle und die Lichtstärke der zweiten Lichtquelle individuell anzusteuern, so dass bei dem gewählten Abstand die voreingestellte relative Beleuchtungsstärke bei dem vorbestimmten Durchmesser vorliegt. Die einzelnen Lichtstärken der Lichtquellen werden individuell so gesteuert, dass der Durchmesser, bei dem eine voreingestellte relative Beleuchtungsstärke des resultierenden Leuchtfelds vorliegt, bei einer nachträglichen Veränderung des vorher gewählten Abstands zwischen dem Leuchtenkörper und der Operationsstelle im Wesentlichen unverändert bleibt.

Alternativ wird bei einer nachträglichen Veränderung des vorher gewählten Abstands zwischen dem Leuchtenkörper und der Operationsstelle das durch eine kippbare Lichtquelle erzeugte Leuchtfeld durch ein Ansteuern eines Kippwinkels der kippbaren Lichtquelle so verlagert, dass der Durchmesser, bei dem eine voreingestellte relative Beleuchtungsstärke des resultierenden Leuchtfelds vorliegt, unverändert bleibt.

Die Erfindung wird nun unter Bezugnahme auf die beigefügten Zeichnungen anhand von Ausführungsbeispielen näher erläutert.

Im Einzelnen zeigen:
- Fig. 1: eine perspektivische Darstellung einer ersten Ausführungsform einer Operationsleuchte;
- Fig. 2: eine Ansicht eines Leuchtenkörpers der ersten Ausführungsform der Operationsleuchte von Fig. 1 von schräg unten;
- Fig. 3a: ein Diagramm einer Lichtverteilung einer Lichtquelle, die ein Leuchtfeld mit einem kleinen Leuchtfelddurchmesser bildet;
- Fig. 3b: ein Diagramm einer Lichtverteilung einer Lichtquelle, die ein Leuchtfeld mit einem großen Leuchtfelddurchmesser bildet;
- Fig. 3c: ein Diagramm einer Lichtverteilung, bei der sich die Leuchtfelder aus Fig. 3a und Fig. 3b überlagern;
- Fig. 3d: ein Diagramm einer Lichtverteilung, bei der sich die Leuchtfelder aus Fig. 3a und Fig. 3b überlagern und bei der die Lichtquellen verschiedene zentrale Beleuchtungsstärken aufweisen;
- Fig. 3e: ein Diagramm mit den überlagerten Lichtverteilungen aus den Fig. 3c und 3d;
- Fig. 4a: eine Schnittdarstellung des Leuchtenkörpers von Fig. 2 entlang der Schnittlinie A-A in Fig. 1 mit einer prinzipiellen Darstellung eines Leuchtfelds einer ersten Lichtquelle;
- Fig. 4b: eine weitere Schnittdarstellung des Leuchtenkörpers von Fig. 2 entlang der Schnittlinie A-A in Fig. 1 mit einer prinzipiellen Darstellung eines Leuchtfelds einer zweiten Lichtquelle;
- Fig. 4c: eine weitere Schnittdarstellung des Leuchtenkörpers von Fig. 2 entlang der Schnittlinie A-A in Fig. 1 mit einer prinzipiellen Darstellung eines resultierenden Leuchtfelds der ersten und zweiten Lichtquelle;
- Fig. 4d: eine weitere Schnittdarstellung des Leuchtenkörpers von Fig. 2 entlang der Schnittlinie A-A in Fig. 1 mit einer prinzipiellen Darstellung eines resultierenden Leuchtfelds der ersten, zweiten, dritten und vierten Lichtquelle im Abstand l₁;
- Fig. 4e: eine weitere Schnittdarstellung des Leuchtenkörpers von Fig. 2 entlang der Schnittlinie A-A in Fig. 1 mit einer prinzipiellen Darstellung des resultierenden Leuchtfeld der ersten, zweiten, dritten und vierten Lichtquelle im Abstand l₂;
- Fig. 5: eine weitere Schnittdarstellung des Leuchtenkörpers von Fig. 2 entlang der Schnittlinie A-A in Fig. 1 mit einer prinzipiellen Darstellung von Leuchtfeldern von zwei ersten Lichtquellen;
- Fig. 6: eine Schnittdarstellung einer weiteren Ausführungsform der Operationsleuchte, mit einer ersten und einer dritten Lichtquelle mit einer prinzipiellen Darstellung von Leuchtfeldern von jeweils zwei ersten und einer dritten Lichtquelle;
- Fig. 7: eine Ausführungsform der Operationsleuchte mit Anbaumodulen;
- Fig. 8: einen Handgriff der Operationsleuchte auf einer Handgriffaufnahme; und
- Fig. 9: eine weitere Ausführungsform der Operationsleuchte, deren Leuchtenkörper unbeweglich befestigt ist.

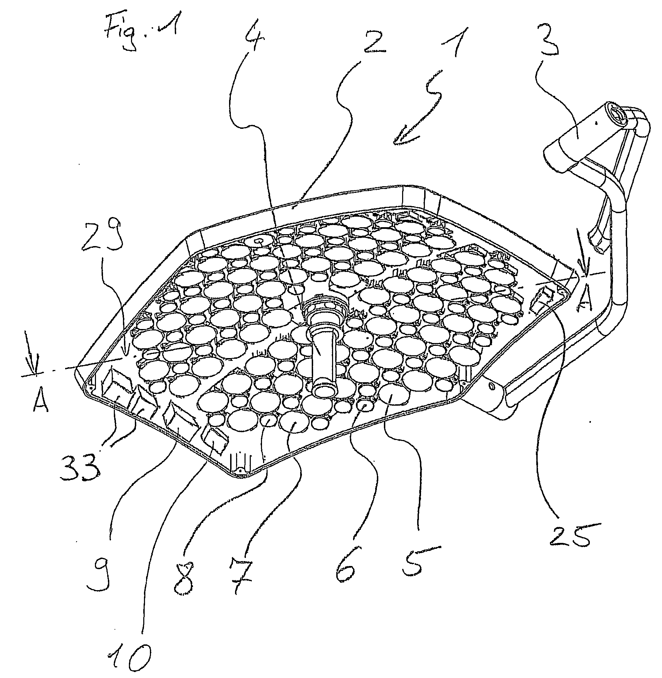

Fig. 1 zeigt eine erste Ausführungsform einer Operationsleuchte 1, die einen Leuchtenkörper 2 und ein Tragsystem 3 aufweist, wobei von dem Tragsystem 3 nur ein so genannter Komfortbügel und ein Teil eines so genannten Viertelbügels gezeigt sind. An dem Leuchtenkörper 2 ist hier im Zentrum des Leuchtenkörpers 2 ein Handgriff 4 angeordnet. Der Handgriff 4 kann in alternativen Ausführungsformen auch an einer anderen Position an dem Leuchtenkörper 2 angeordnet sein. In dem Leuchtenkörper 2 sind erste Lichtquellen 5, zweite Lichtquellen 6, dritte Lichtquellen 7 und vierte Lichtquellen 8 angeordnet. Weiterhin nimmt der Leuchtenkörper 2 eine Steuerungsvorrichtung 9 auf, wobei die Steuerungsvorrichtung 9 in alternativen Ausführungsformen auch in einem separaten Gehäuse und/oder an einer anderen Position der Operationsleuchte 1 oder ihrer Umgebung vorgesehen sein kann.

Im Leuchtenkörper 2 ist außerdem ein Mittel 25 zum Auslösen der Änderung der individuellen Lichtstärken der Lichtquellen zum Anpassen eines nachstehend beschriebenen resultierenden Leuchtfelds, beispielsweise ein Bewegungssensor oder Beschleunigungssensor, vorgesehen, der mit der Steuerungsvorrichtung 9 verbunden ist. Mit dem Bewegungssensor wird eine Bewegung des Leuchtenkörpers 2 erfasst und nach Beendigung der Bewegung wird ein entsprechendes Signal an die Steuerungsvorrichtung 9 gegeben. Eine Bewegung kann aber auch alternativ auf eine andere Weise, beispielsweise durch eine Auswertung von Signalen einer Abstandsmessvorrichtung, erfasst werden. Nach Beendigung der Bewegung führt die Steuerungsvorrichtung 9 dann eine Anpassung des resultierenden Leuchtfelds durch. Alternativ kann das Auslösen auch manuell, beispielsweise durch Betätigen oder Loslassen eines Schalters oder Sensors, erfolgen.

Des Weiteren sind in dem Leuchtenkörper 2 Ansteuereinheiten 33 für jede Lichtquelle 5, 6, 7, 8 zum Ansteuern ihrer Lichtstärke vorgesehen.

Das Tragsystem 3 ermöglicht es, den Leuchtenkörper 2 innerhalb eines festgelegten räumlichen Bewegungsbereichs an einer beliebigen Position in einer beliebigen Orientierung zu positionieren, um eine Operationsstelle eines Patienten möglichst optimal auszuleuchten.

Die Lichtquellen 5, 6, 7, 8 enthalten LEDs mit einer optischen Vorrichtung, um die Lichtstrahlen der LEDs jeweils zu einem Lichtbündel zu bündeln. Um eine geeignete Farbtemperatur bei einer guten Farbwiedergabe zu erzielen, können LEDs mit verschiedenen weißen Farbtönen (warmweiß und kaltweiß) verwendet werden. Dadurch kann auch eine Farbtemperatur des von der Operationsleuchte 1 abgestrahlten Lichts eingestellt werden. Für einen größeren Einstellbereich der Farbtemperatur können in alternativen Operationsleuchten auch farbige LEDs verwendet werden. Alternativ können Lichtquellen, die Licht mit der gleichen Farbtemperatur abgeben, verwendet werden.

Die optischen Vorrichtungen sind hier Linsen, wobei zwei unterschiedliche Typen von Linsen eingesetzt werden, die das abgestrahlte Licht der LEDs so lenken, dass Lichtbündel erzeugt werden. Ein Linsentyp, hier ein Typ mit einem großen Linsendurchmesser in den Lichtquellen 5, 7, erzeugt ein Lichtbündel, das ein Leuchtfeld mit einem kleinen Durchmesser erzeugt, ein anderer Linsentyp, hier mit ein Typ mit einem kleinen Linsendurchmesser in den Lichtquellen 6, 8, erzeugt ein Lichtbündel, das ein Leuchtfeld mit einem großen Leuchtfelddurchmesser erzeugt. Alternativ ist auch die Verwendung von Linsen möglich, die den selben Durchmesser aufweisen aber unterschiedliche optische Eigenschaften haben. Die durch die verschiedenen Linsen erzeugten Leuchtfelder weisen auf Grund von unterschiedlichen optisch wirksamen Flächen der Linsen und/oder den unterschiedlichen Durchmessern der Linsen unterschiedliche Lichtverteilungen und unterschiedliche Leuchtfelddurchmesser auf. In weiteren alternativen Ausführungsformen ist der Einsatz von anderen Mitteln zur Erzeugung von Lichtbündeln, die Leuchtfelder mit verschiedenen Durchmessern erzeugen, wie z.B. Reflektoren, denkbar.

In einer ersten Ausführungsform sind nur die ersten Lichtquellen 5 und zweiten Lichtquellen 6 vorgesehen, die in dem Leuchtenkörper 2 starr angebracht sind.

In einer zweiten Ausführungsform sind die Lichtquellen 5, 6 analog zu den später beschriebenen Lichtquellen 7, 8 kippbar gelagert und alternativ einzeln, in Gruppen oder gemeinsam mit einer Antriebsvorrichtung 10 zum Kippen versehen.

Wie später beschrieben, ist es zur Erzeugung von verschiedenen Leuchtfelddurchmessern in verschiedenen Abständen zum Leuchtenkörper 2 günstig, aber nicht zwingend, in einer dritten Ausführungsform zusätzlich zu den starren Lichtquellen weitere Lichtquellen vorzusehen, die kippbar in dem Leuchtenkörper 2 angeordnet sind. Hierfür sind die Lichtquellen 7, 8 vorgesehen, die entsprechend kippbar gelagert sind, und für die in dem Leuchtenkörper 2 die Antriebsvorrichtung 10 zum Kippen der Lichtquellen 7, 8 alternativ einzeln, in Gruppen oder gemeinsam vorgesehen ist. Die Lichtquellen 7 entsprechen bis auf die Kippbarkeit den Lichtquellen 5, und die Lichtquellen 8 entsprechen bis auf die Kippbarkeit den Lichtquellen 6.

Die Lichtquellen 5, 6 sind so angeordnet, dass die Lichtaustrittsflächen sämtlicher Linsen in einer sphärischen Fläche mit einem Radius von 1300 mm angeordnet sind. In alternativen Ausführungsformen können die Lichtquellen auch so angeordnet sein, dass die Lichtaustrittsflächen nicht in einer Fläche angeordnet sind, dass die Fläche nicht sphärisch ist, oder die sphärische Fläche einen anderen Radius aufweist.

Die Lichtquellen 5, 6, 7, 8 sind jeweils über die Ansteuereinheiten 33 mit der Steuerungsvorrichtung 9 verbunden, und die Lichtstärken der einzelnen Lichtquellen 5, 6, 7, 8 werden von der Steuerungsvorrichtung 9 angesteuert. Die Antriebsvorrichtung 10 zum Kippen der Lichtquellen 7 und 8 ist ebenfalls mit der Steuerungsvorrichtung 9 verbunden und ein entsprechender durch die Antriebsvorrichtung 10 einstellbarer Kippwinkel der Lichtquellen 7, 8 ist durch die Steuerungsvorrichtung 9 ansteuerbar.

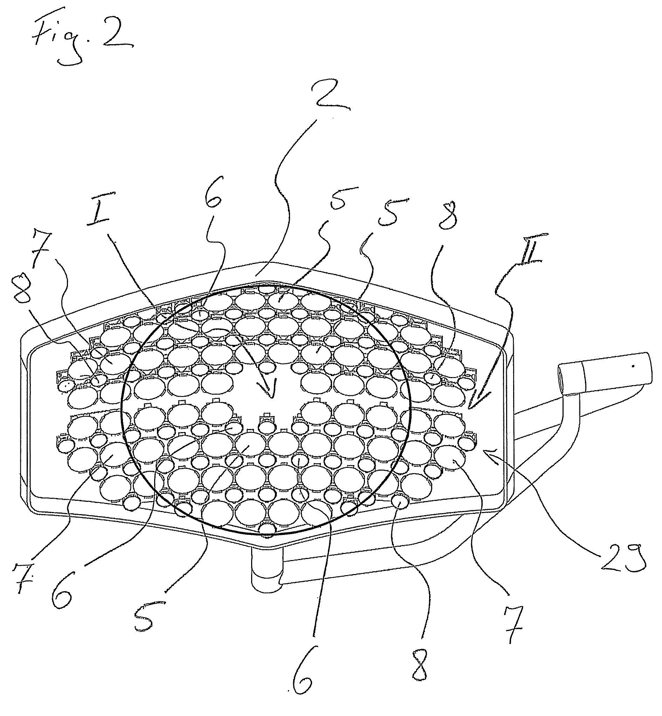

Fig. 2 zeigt den Leuchtenkörper 2 der dritten Ausführungsform, ohne Handgriff dargestellt, von schräg unten. Hierbei ist zu erkennen, dass der Leuchtenkörper 2 eine Lichtaustrittsfläche 29 aufweist, und die Lichtaustrittsfläche 29 in einen kreisförmigen inneren Bereich I und einen darum herum angeordneten äußeren Bereich II unterteilt ist. Die Lichtquellen 5, 6, die sich zumindest in dem Bereich I befinden, sind starr angeordnet, und die Lichtquellen 7, 8, die sich zumindest im Bereich II befinden, also einen größeren Abstand zu einer später gezeigten optischen Achse des Leuchtenkörpers 2 haben, sind kippbar in dem Leuchtenkörper 2 befestigt.

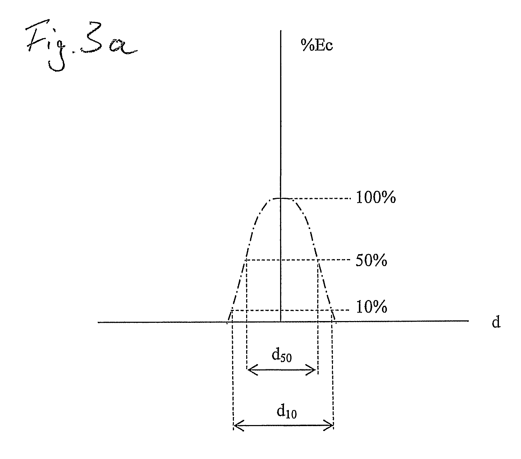

In den Figuren 3a bis 3d wird das Prinzip der Veränderung des Leuchtfelddurchmessers durch unterschiedliches Ansteuern der Lichtquellen erklärt.

Unter Leuchtfeld versteht man den beleuchteten Bereich auf einer Operationsstelle. In Fig. 3a ist beispielsweise in einem Diagramm eine normgerechte Lichtverteilung in dem Leuchtfeld einer Operationsleuchte gezeigt. Die Lichtverteilung im Leuchtfeld ist gemäß der derzeit geltenden Norm DIN EN 60601-2-41:2010 wie folgt definiert: Das Leuchtfeld weist eine zentrale Beleuchtungsstärke von 100% E_{C} auf. Der Durchmesser eines Kreises um das Leuchtfeldzentrum, auf dem die Beleuchtungsstärke 10% der zentralen Beleuchtungsstärke E_{C} beträgt, ist der Leuchtfelddurchmesser. Dieser Leuchtfelddurchmesser wird als "d₁₀" bezeichnet. Ein weiterer Kennwert des Leuchtfelds ist ein Durchmesser, bei dem die Beleuchtungsstärke 50% der zentralen Beleuchtungsstärke E_{C} beträgt. Dieser Durchmesser wird auch als "d₅₀" bezeichnet. Der Durchmesser d₅₀ ist gemäß der Norm so festgelegt, dass er mindestens die Hälfte des Leuchtfelddurchmessers d₁₀ betragen muss. Diese Bedingungen müssen bei einem Abstand von 1000 mm zwischen der Lichtaustrittsfläche und dem Leuchtfeld eingehalten werden.

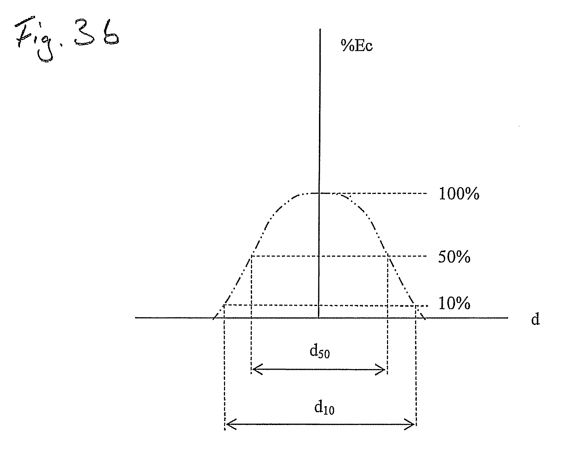

In Fig. 3a ist mit einer Strich-Punkt-Linie die Lichtverteilung einer Lichtquelle gezeigt, die prinzipiell der Lichtquelle 5 in den Ausführungsbeispielen entspricht. In Fig. 3b ist mit einer Strich-Zweipunkt-Linie die Lichtverteilung einer Lichtquelle gezeigt, die prinzipiell der Lichtquelle 6 in den Ausführungsbeispielen entspricht. Der Leuchtfelddurchmesser d₁₀ der Lichtquelle 5 (Fig. 3a) ist kleiner als der Leuchtfelddurchmesser d₁₀ der Lichtquelle 6 (Fig. 3b).

Als gestrichelte horizontale Linien sind in den Figuren 3a und 3b die relativen Beleuchtungsstärken 10%, 50% und 100% der jeweiligen Lichtquellen eingetragen. Aus den Schnittpunkten der gestrichelten horizontalen Linien und der Linie, die die Lichtverteilung angibt, ergeben sich der Durchmesser d₅₀ und der Leuchtfelddurchmesser d₁₀. Wie aus den Diagrammen zu erkennen ist, erfüllen die Leuchtfelder die Normenanforderung hinsichtlich der Lichtverteilung, da der Durchmesser d₅₀ mehr als die Hälfte des Leuchtfelddurchmessers d₁₀ beträgt.

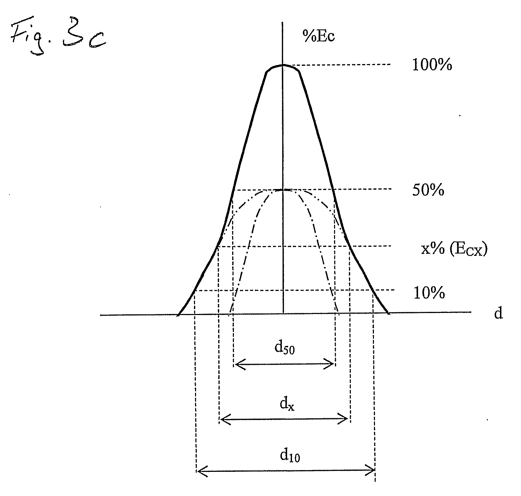

In Fig. 3c ist mit den identischen Linientypen jeweils die Lichtverteilung aus den Fig. 3a und 3b übertragen und mit einer durchgezogenen Linie ist die Lichtverteilung in einem resultierenden Leuchtfeld gezeigt, das von den beiden Lichtquellen aus Fig. 3a und Fig. 3b durch Überlagerung des Lichts gebildet wird. Durch die Überlagerung addieren sich die jeweiligen Beleuchtungsstärken auf den identischen Durchmessern.

In der in der Fig. 3c gezeigten Situation ist die zentrale Beleuchtungsstärke E_{C} der einzelnen Lichtquellen gleich groß und beträgt deshalb jeweils 50% der zentralen Beleuchtungsstärke E_{C} des resultierenden Leuchtfelds. Als gestrichelte horizontale Linien sind hier die Beleuchtungsstärken eingetragen, die 10% bzw. 50% bzw. 100% der zentralen Beleuchtungsstärke E_{C} des resultierenden Leuchtfelds betragen. Anhand der Schnittpunkte dieser horizontalen Linien mit der Linie der Lichtverteilung des resultierenden Leuchtfelds ergeben sich jeweils die Durchmesser, auf denen die Beleuchtungsstärken 10% bzw. 50% der zentralen Beleuchtungsstärke E_{C} des resultierenden Leuchtfelds betragen.

Darüber hinaus ist in der Fig. 3c eine relative Beleuchtungsstärke x% der zentralen Beleuchtungsstärke E_{C} eingetragen, die beliebig entweder werksseitig oder vom Nutzer festgelegt und voreingestellt werden kann. Hierbei ergibt sich dann durch die Schnittpunkte mit der Linie der Beleuchtungsstärke des resultierenden Leuchtfelds der Durchmesser dₓ eines Kreises, auf dem die Beleuchtungsstärke x% der zentralen Beleuchtungsstärke E_{C} des resultierenden Leuchtfelds beträgt. Der Durchmesser dₓ kann, wie später beschrieben, als ein Durchmesser, auf dem eine beliebige voreingestellte relative Beleuchtungsstärke E_{CX} des resultierenden Leuchtfelds vorliegt, konstant gehalten werden. Üblich ist, dass der Leuchtfelddurchmesser d₁₀ als der Durchmesser dₓ festgelegt wird, der bei einer Veränderung des Abstands unverändert bleiben soll.

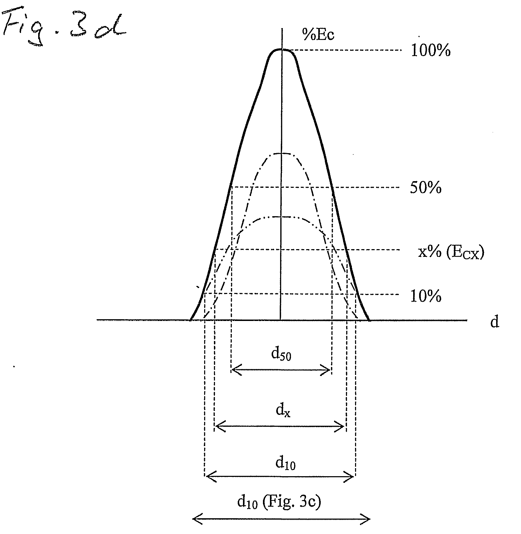

Fig. 3d zeigt die Lichtverteilung, wenn die einzelnen Lichtquellen so angesteuert werden, dass unterschiedliche zentrale Beleuchtungsstärken E_{C} der einzelnen Lichtquellen vorliegen. In diesem Fall ist die zentrale Beleuchtungsstärke E_{C} der Lichtquelle, die das Leuchtfeld mit dem kleineren Leuchtfelddurchmesser erzeugt (Strich-Zweipunkt-Linie; Fig. 3b) geringer als in der in Fig. 3c gezeigten Situation. Die zentrale Beleuchtungsstärke E_{C} der Lichtquelle, die das Leuchtfeld mit dem größeren Durchmesser erzeugt (Strich-Punkt-Linie; Fig. 3a) ist größer als in der in Fig. 3c gezeigten Situation.

Wie aus den Diagrammen der Fig. 3c und 3d zu erkennen ist, erfüllen auch die resultierenden Leuchtfelder die Normenanforderung hinsichtlich der Lichtverteilung, da der Durchmesser d₅₀ mehr als die Hälfte des Leuchtfelddurchmessers d₁₀ beträgt.

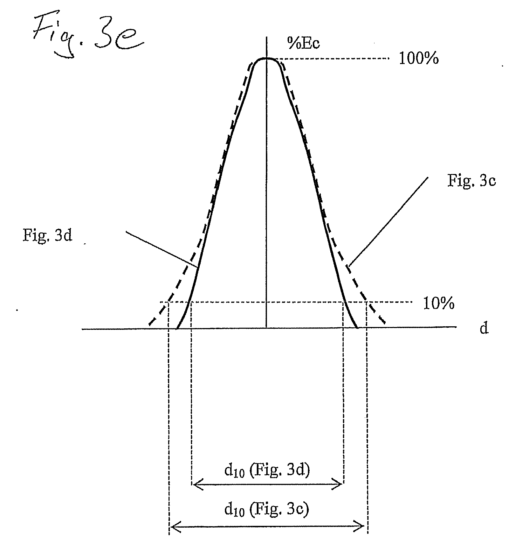

Anhand des in Fig. 3e gezeigten Vergleichs der Leuchtfelddurchmesser d₁₀ der resultierenden Leuchtfelder aus Fig. 3c und Fig. 3d lässt sich erkennen, dass bei einer Erhöhung der Beleuchtungsstärke durch die Lichtquelle, die einen kleineren Leuchtfelddurchmesser d₁₀ erzeugt, und einer Verringerung der Beleuchtungsstärke durch die Lichtquelle, die einen größeren Leuchtfelddurchmesser d₁₀ erzeugt, der Leuchtfelddurchmesser d₁₀ des resultierenden Leuchtfelds kleiner wird.

Grundsätzlich wird ein kleinerer Leuchtfelddurchmesser d₁₀ erzeugt, wenn die Beleuchtungsstärke der Lichtquelle, die einen kleineren Leuchtfelddurchmesser d₁₀ erzeugt, erhöht wird und/oder die Beleuchtungsstärke der Lichtquelle, die einen größeren Leuchtfelddurchmesser d₁₀ erzeugt, verringert wird.

Analog wird ein größerer Leuchtfelddurchmesser d₁₀ erzeugt, wenn die Beleuchtungsstärke der Lichtquelle, die einen kleineren Leuchtfelddurchmesser d₁₀ erzeugt, verringert wird und/oder die Beleuchtungsstärke der Lichtquelle, die einen größeren Leuchtfelddurchmesser d₁₀ erzeugt, erhöht wird.

Um die zentrale Beleuchtungsstärke E_{C} des resultierenden Leuchtfelds konstant zu halten, kann die zentrale Beleuchtungsstärke E_{C} einer der Lichtquellen um den gleichen Betrag verringert werden, um den die zentrale Beleuchtungsstärke E_{C} der anderen Lichtquelle erhöht wird, so dass die Summe der einzelnen zentralen Beleuchtungsstärken E_{C} gleich bleibt.

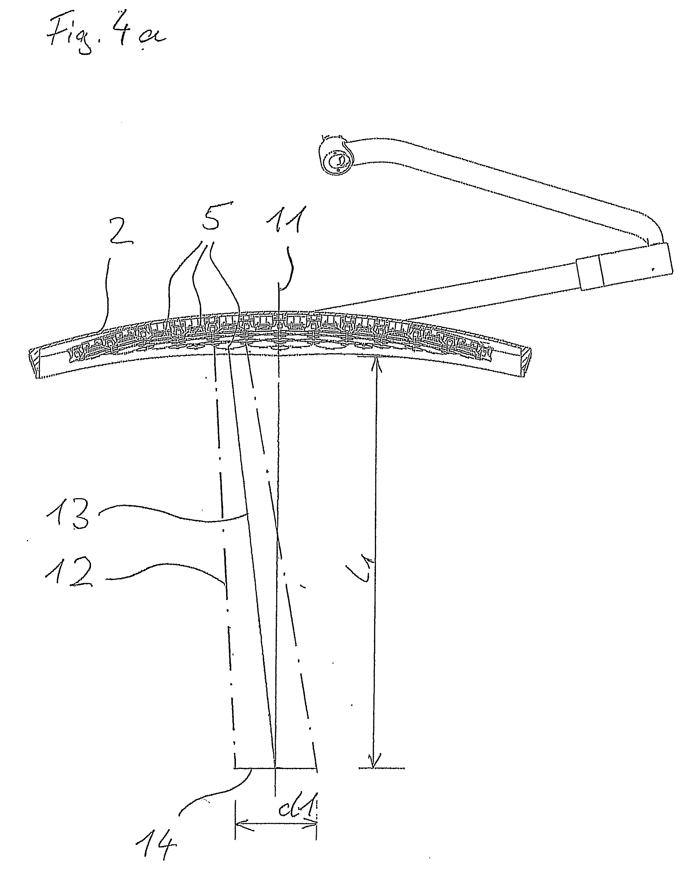

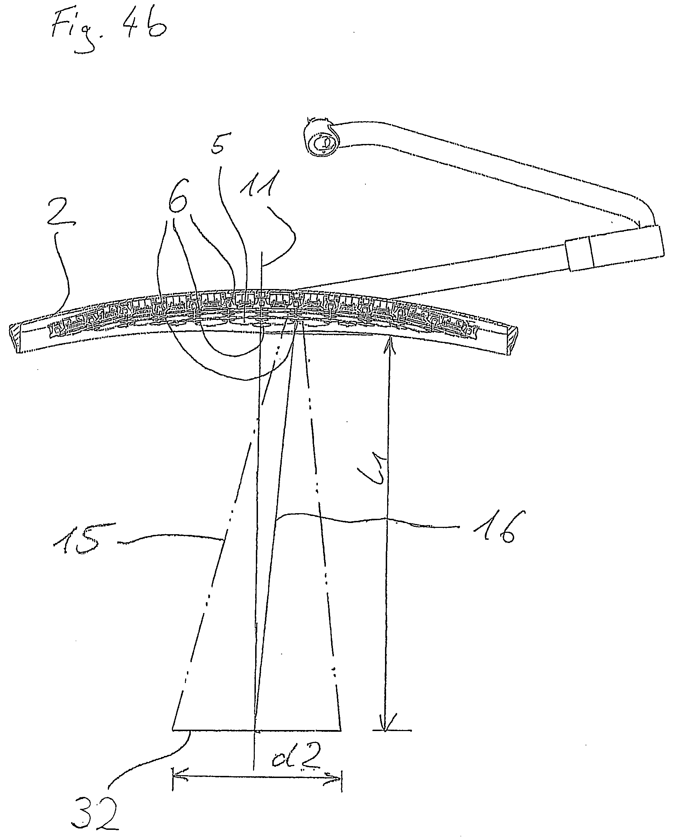

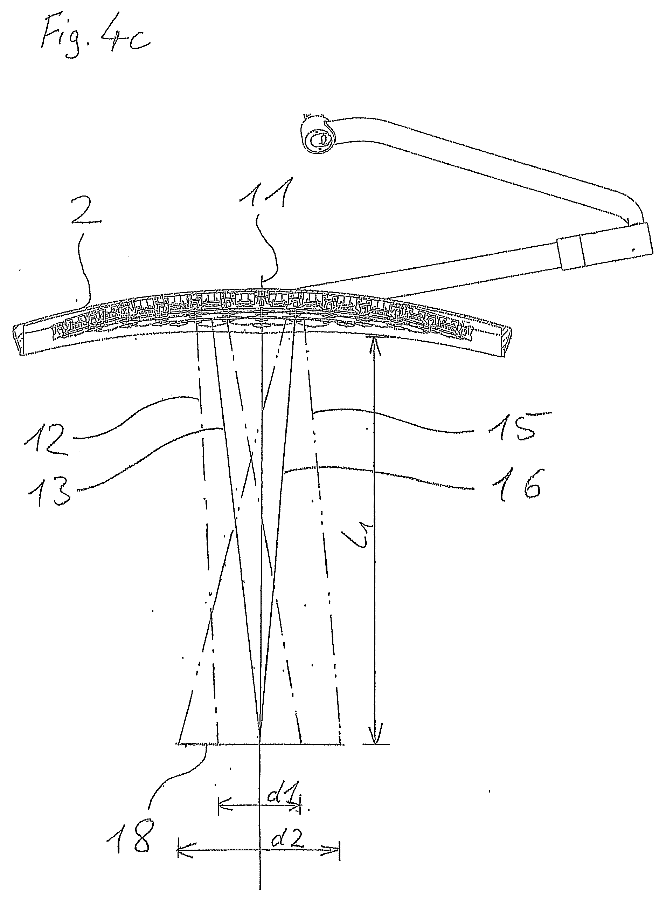

In den Fig. 4a bis 4d wird prinzipiell gezeigt, wie Leuchtfelder durch Überlagerung von verschiedenen Lichtbündeln erzeugt werden. Die Darstellung der verschiedenen Lichtbündel ist nicht so zu verstehen, dass die Darstellung eine exakte Hell-Dunkel-Grenze darstellt. Auch die Darstellung von verschiedenen Durchmessern stellt keine exakte Hell-Dunkel-Grenze dar, da bei der Definition des Durchmessers als Leuchtfelddurchmesser d₁₀ (Fig. 4a, 4b, 4c) auch außerhalb dieses Durchmessers Licht mit einem Anteil von kleiner als 10% der zentralen Beleuchtungsstärke E_{c} (Streulicht) vorhanden sein kann.

In Fig. 4a ist eine Schnittdarstellung des Leuchtenkörpers 2 entlang einer Schnittlinie A-A in Fig. 1 gezeigt. Der Leuchtenkörper 2 weist eine optische Achse 11 auf, auf der sich der Mittelpunkt eines erzeugten Leuchtfelds befindet.

Die Lichtquellen 5 strahlen jeweils ein Lichtbündel 12 ab, das durch eine Strichpunktlinie dargestellt ist, wobei hier aus Gründen der Übersichtlichkeit nur eines der Lichtbündel 12 gezeigt ist. Das Lichtbündel 12 hat eine Achse 13, die sich in einem Abstand l₁ von dem Leuchtenkörper 2 mit dessen optischer Achse 11 schneidet. Die weiteren Lichtquellen 5 sind in dem Leuchtenkörper 2 so verteilt angeordnet, dass die Achsen ihrer Lichtbündel die optische Achse 11 in dem selben Punkt wie die Achse 13 der Lichtquelle 5 schneiden, so dass sämtliche Lichtquellen 5 ein Leuchtfeld 14 bilden.

Der Abstand l₁ ist in dieser Ausführungsform auf 1300 mm festgelegt, kann aber in anderen Ausführungsformen abhängig von dem Verwendungszweck und der Leuchtenkörpergröße auf einen anderen Wert festgelegt werden.

Im Abstand l₁ von dem Leuchtenkörper 2 wird das erste Leuchtfeld 14 auf der Operationsstelle gebildet. Das erste Leuchtfeld 14, das durch die Lichtbündel der ersten Lichtquellen 5 gebildet wird, weist einen Leuchtfelddurchmesser d₁₀ auf, der mit d1 bezeichnet ist.

Auch in Fig. 4b ist eine Schnittdarstellung des Leuchtenkörpers 2 entlang der Schnittlinie A-A in Fig. 1 gezeigt. Der Leuchtenkörper 2 weist auch hier die optische Achse 11 auf.

Die Lichtquellen 6 strahlen jeweils ein Lichtbündel 15 ab, das durch eine Strich-Zweipunkt-Linie dargestellt ist, wobei auch hier aus Gründen der Übersichtlichkeit auch nur eines der Lichtbündel 15 gezeigt ist. Das Lichtbündel 15 hat eine Achse 16, die sich in dem Abstand l₁ von dem Leuchtenkörper 2 mit dessen optischer Achse 11 schneidet. Die weiteren Lichtquellen 6 sind in dem Leuchtenkörper 2 so verteilt angeordnet, dass die Achsen ihrer Lichtbündel die optische Achse 11 in dem selben Punkt wie die Achse 16 der gezeigten Lichtquelle 6 schneiden, so dass sämtliche Lichtquellen 6 ein zweites Leuchtfeld 32 bilden.

Der Abstand l₁ ist auch hier auf 1300 mm festgelegt, kann aber in anderen Ausführungsformen abhängig von dem Verwendungszweck und der Leuchtenkörpergröße auf einen anderen Wert bestimmt werden.

Im Abstand l₁ von dem Leuchtenkörper 2 wird durch die Lichtbündel 15 der zweiten Lichtquellen 6 das zweite Leuchtfeld 32 auf der Operationsstelle gebildet. Das zweite Leuchtfeld 32 weist einen Leuchtfelddurchmesser d₁₀ auf, der mit d2 bezeichnet ist.

Der Durchmesser d2 des durch die zweiten Lichtquellen 6 gebildeten zweiten Leuchtfelds 32 ist größer als der Durchmesser d1 des

Der Durchmesser d2 des durch die zweiten Lichtquellen 6 gebildeten zweiten Leuchtfelds 32 ist größer als der Durchmesser d1 des durch die ersten Lichtquellen 5 gebildeten ersten Leuchtfelds 14 (siehe Fig. 4a).

Fig. 4c zeigt die überlagerten Lichtbündel 12 und 15, die in den Figuren 4a und 4b beschrieben wurden.

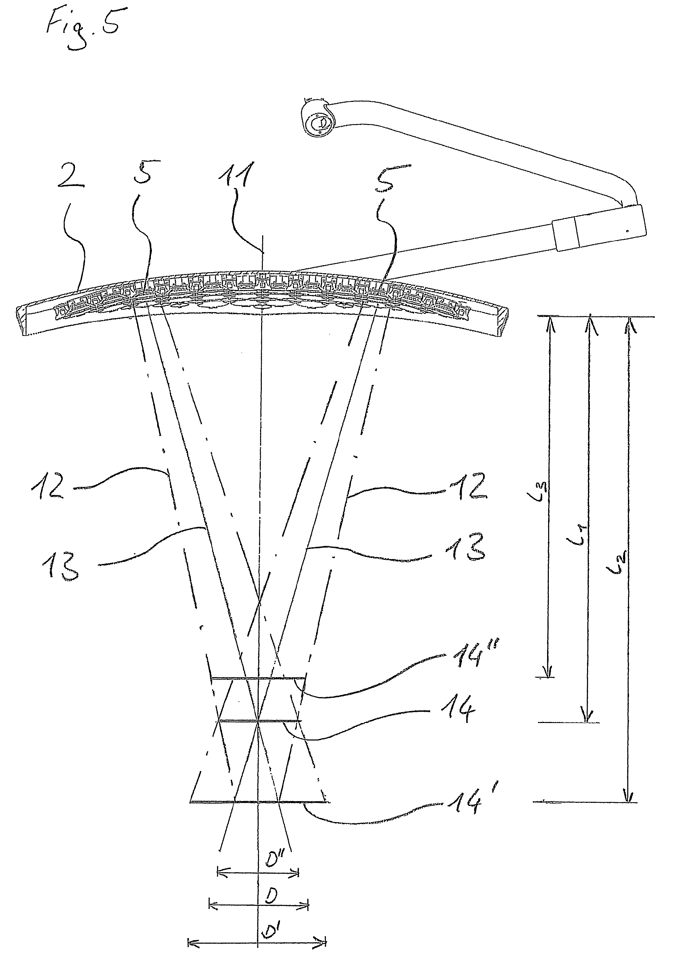

Durch die Überlagerung der Lichtbündel 12, 15 bilden die erste Lichtquelle 5 und die zweite Lichtquelle 6 auf der Operationsstelle, die sich in dem Abstand l₁ von dem Leuchtenkörper 2 befindet, auf die zu den Fig. 3c, 3d und 5 beschriebene Art und Weise, ein resultierendes Leuchtfeld 18. Das Leuchtfeld mit dem kleineren Durchmesser d1, das durch das Lichtbündel 12 gebildet wird, und das Leuchtfeld mit dem größeren Durchmesser d2, das durch das Lichtbündel 15 gebildet wird, werden dabei überlagert.

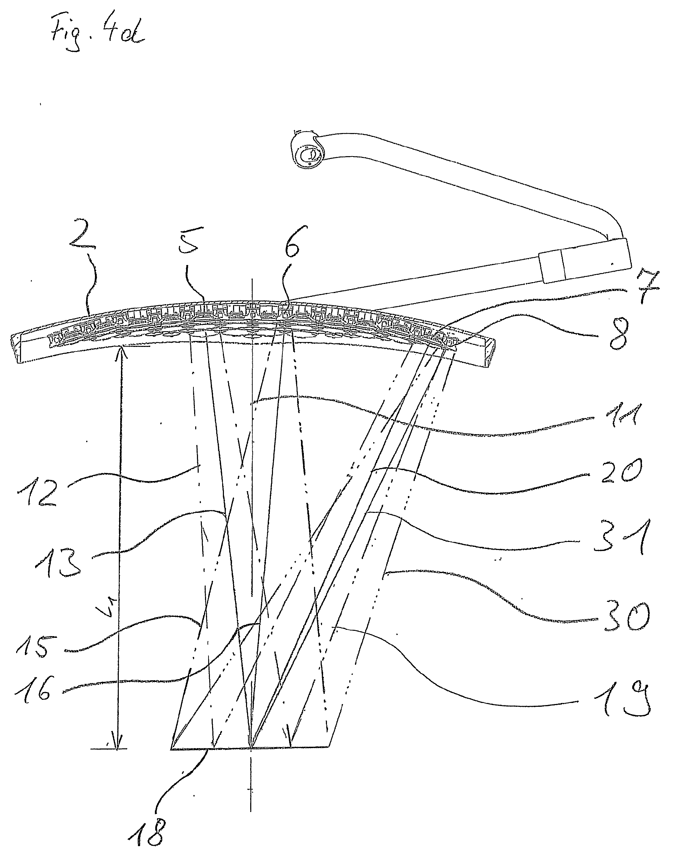

In Fig. 4d ist ein exemplarisches optionales drittes Lichtbündel 19 gezeigt, das mit einer Strich-Drei-Punkt-Linie dargestellt ist. Das dritte Lichtbündel 19 wird von der dritten Lichtquelle 7 ausgestrahlt, wobei auch hier aus Gründen der Übersichtlichkeit nur eines der dritten Lichtbündel 19 gezeigt ist. Die weiteren Lichtquellen 7 sind in dem Leuchtenkörper 2 so angeordnet, dass die Achsen ihrer Lichtbündel die optische Achse 11 in dem selben Punkt wie eine Achse 20 der gezeigten Lichtquelle 7 schneiden. Ein exemplarisches optionales viertes Lichtbündel 30, das mit einer Strich-Vier-Punkt-Linie dargestellt ist, wird von der vierten Lichtquelle 8 ausgestrahlt, wobei auch hier aus Gründen der Übersichtlichkeit nur eines der vierten Lichtbündel 30 gezeigt ist. Die weiteren vierten Lichtquellen 8 sind in dem Leuchtenkörper 2 so angeordnet, dass die Achsen ihrer Lichtbündel die optische Achse 11 in dem selben Punkt wie eine Achse 31 der Lichtquelle 8 schneiden. Das dritte Lichtbündel 19 weist die Achse 20 auf, und das vierte Lichtbündel 30 weist die Achse 31 auf, die sich mit der optischen Achse 11 des Leuchtenkörpers 2 bei dieser Einstellung des Kippwinkels der dritten und vierten Lichtquellen 7, 8 in dem selben Schnittpunkt schneiden, wie die Achsen 13 der Lichtbündel 12 der ersten Lichtquellen 5, und die Achsen 16 der Lichtbündel 15 der zweiten Lichtquellen 6, hier im Abstand l₁. Das dritte Lichtbündel 19 erzeugt ein Leuchtfeld mit einem kleineren Durchmesser und das vierte Lichtbündel 30 erzeugt ein Leuchtfeld mit einem größeren Durchmesser und gemeinsam mit dem ersten Lichtbündel 12 und dem zweiten Lichtbündel 15 erzeugen das dritte Lichtbündel 19 und das vierte Lichtbündel 20 auf die zu den Fig. 3c, 3d und 5 beschriebene Art und Weise das resultierende Leuchtfeld 18. Die Leuchtfelder mit den kleineren Durchmessern, die durch die Lichtbündel 12 und 19 gebildet werden, und die Leuchtfelder mit dem größeren Durchmesser, die durch die Lichtbündel 15 und 30 gebildet werden, werden dabei überlagert.

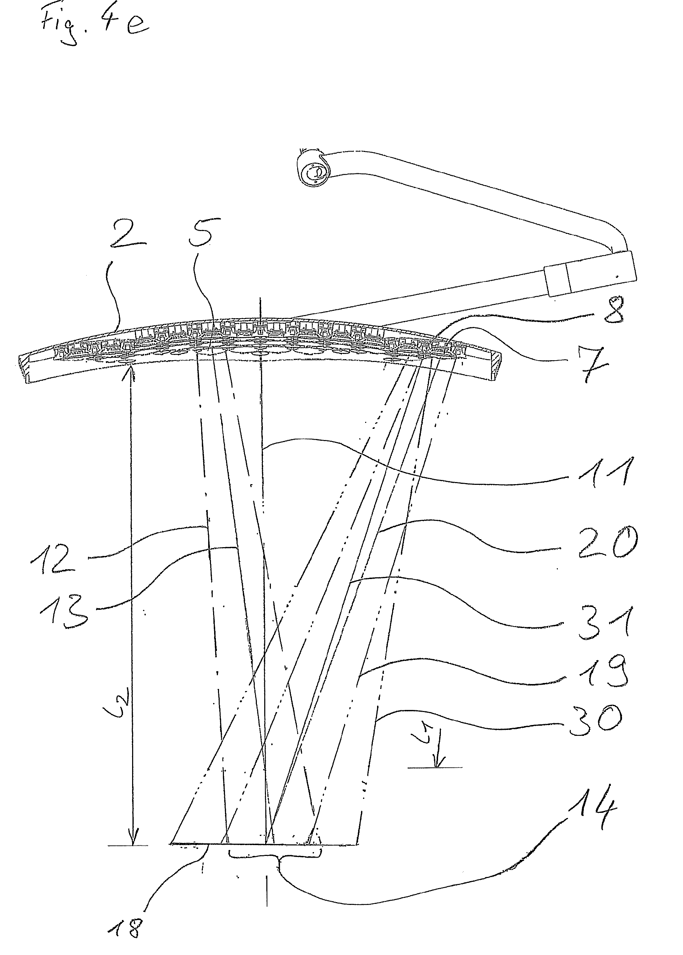

Fig. 4e zeigt eine Situation, in der der Abstand der Operationsstelle, auf der ein Leuchtfeld gebildet wird, größer, nämlich l₂, ist. Daher werden die Leuchtfelder im Abstand l₂ abgebildet.

Das gezeigte Lichtbündel 12 von einer der ersten Lichtquellen 5 wird dann in dem ersten Leuchtfeld 14 abgebildet, das nicht konzentrisch zu der optischen Achse 11 ist, da sich seine Achse 13 im Abstand l₁ mit der optischen Achse 11 schneidet und sich die Achse 13 im Abstand l₂ nicht mit der optischen Achse 11 schneidet. Durch die Überlagerung von mehreren Lichtbündeln 12, ausgehend von über den Umfang des Leuchtenkörpers 2 verteilten Lichtquellen 5, entsteht jedoch wieder ein Leuchtfeld aus den Lichtbündeln 12 der ersten Lichtquellen 5, das konzentrisch zu der optischen Achse 11 ist.

Die dritten Lichtquellen 7 und die vierten Lichtquellen 8 werden mit Hilfe der Steuerungsvorrichtung 9, die die Antriebsvorrichtung 10 ansteuert, so gekippt, dass sich die Achsen 20, 31 der Lichtbündel 19, 30 in dem Abstand l₂ auf der optischen Achse 11 schneiden. Nach einer Veränderung des Abstands durch Bewegen des Leuchtenkörpers 2 werden die dritten Lichtquellen 7 und die vierten Lichtquellen 8 durch die Antriebsvorrichtung 10 so gekippt, dass sich der Schnittpunkt der Achsen 20 und 31 jeweils in dem tatsächlichen Abstand der Operationsstelle von dem Leuchtenkörper 2 befindet.

In Fig. 5 ist eine Schnittdarstellung des Leuchtenkörpers 2 mit einer prinzipiellen Darstellung von drei Leuchtfeldern 14, 14', 14'', die von zwei der ersten Lichtquellen 5 erzeugt werden. Wie aus Fig. 5 zu ersehen ist, wird das Leuchtfeld 14 auf einer Operationsstelle im Abstand l₁, das Leuchtfeld 14' auf einer Operationsstelle im Abstand l₂ und das Leuchtfeld 14" auf einer Operationsstelle im Abstand l₃ erzeugt.

Der Leuchtenkörper 2 weist auch hier die optische Achse 11 auf, auf der sich die Mittelpunkte der erzeugten Leuchtfelder befinden. Die Lichtquellen 5 strahlen jeweils das Lichtbündel 12 ab, wobei hier aus Gründen der Übersichtlichkeit nur zwei der Lichtbündel 12 gezeigt sind. Die Lichtbündel 12 haben auch hier jeweils die Achse 13, die sich in dem Abstand l₁ von dem Leuchtenkörper 2 mit dessen optischer Achse 11 schneiden. Die weiteren Lichtquellen 5 sind in dem Leuchtenkörper 2 so verteilt angeordnet, dass die Achsen ihrer Lichtbündel die optische Achse 11 in dem selben Punkt wie die Achse 13 der Lichtquelle 5 schneiden, so dass sämtliche Lichtquellen 5 das Leuchtfeld 14 auf der Operationsstelle im Abstand l₁ bilden.

Aus der Fig. 5 ist prinzipiell ersichtlich, dass die Durchmesser D, D', D" abhängig von den Abständen l₁, l₂ und l₃ der Leuchtfelder 14, 14', 14" von dem Leuchtenkörper 2 unterschiedlich groß sind. Das Leuchtfeld 14, das auf der Operationsstelle im Abstand l₁, in dem sich die Achsen 13 mit der optischen Achse 11 schneiden, gebildet wird, weist den kleinsten Durchmesser D auf. Bei einer Veränderung des Abstands der Operationsstelle beispielsweise auf l₂ oder l₃ werden die Leuchtfelder 14', 14" erzeugt, die die größeren Durchmesser D', D" aufweisen. Die Durchmesser D, D', D" sind nur prinzipiell dargestellt und stellen nicht zwingend die Leuchtfelddurchmesser d₁₀ oder die Durchmesser dₓ, bei dem eine relative Beleuchtungsstärke E_{cx} vorliegt, der Leuchtfelder 14, 14', 14" dar. Durch diese Darstellung wird lediglich gezeigt, dass sich prinzipiell der Durchmesser eines Leuchtfelds ändert, wenn der Abstand zwischen dem Leuchtenkörper und der Operationsstelle verändert wird.

Wie die prinzipiell dargestellten Durchmesser D, D', D" der Leuchtfelder 14, 14', 14" ändern sich bei einer Abstandsänderung auch sowohl der Leuchtfelddurchmesser d₁₀ als auch der Durchmesser dₓ, bei dem eine relative Beleuchtungsstärke E_{cx} vorliegt.

Um bei einer Veränderung des Abstands von l₁ auf l₂ oder l₃ die Vergrößerung des Leuchtfelddurchmessers d₁₀ oder des Durchmessers dₓ, bei dem die relative Beleuchtungsstärke E_{cx} vorliegt, zu verhindern, ist es erforderlich, die Lichtquelle 5, die das Leuchtfeld mit dem kleineren Leuchtfelddurchmesser d1 erzeugt (Fig. 4a, 4c), so anzusteuern, dass dessen Lichtstärke erhöht wird und/oder die Lichtquelle 6, die das Leuchtfeld mit dem größeren Leuchtfelddurchmesser d2 erzeugt (Fig. 4b, 4c), so anzusteuern, dass dessen Lichtstärke verringert wird.

Umgekehrt ist es bei einer Abstandsänderung von den Abständen l₂ oder l₃ auf l₁ erforderlich, die Verkleinerung des Leuchtfelddurchmessers d₁₀ oder des Durchmessers dₓ, bei dem die relative Beleuchtungsstärke E_{cx} vorliegt, zu verhindern. Dazu ist es erforderlich, die Lichtquelle 5, das das Leuchtfeld mit dem kleineren Leuchtfelddurchmesser d1 erzeugt (Fig. 4a, 4c), so anzusteuern, dass dessen Lichtstärke verringert wird und/oder die Lichtquelle 6, die das Leuchtfeld mit dem größeren Leuchtfelddurchmesser d2 erzeugt (Fig. 4b, 4c), so anzusteuern, dass dessen Lichtstärke erhöht wird.

Nach der Ansteuerung der Lichtquellen 5, 6, so dass eine Veränderung des Leuchtfelddurchmessers d₁₀ verhindert wird, wird dann gegebenenfalls die Ansteuerung der Lichtquellen so angepasst, dass wieder die vorherige zentrale Beleuchtungsstärke E_{c} vorliegt.

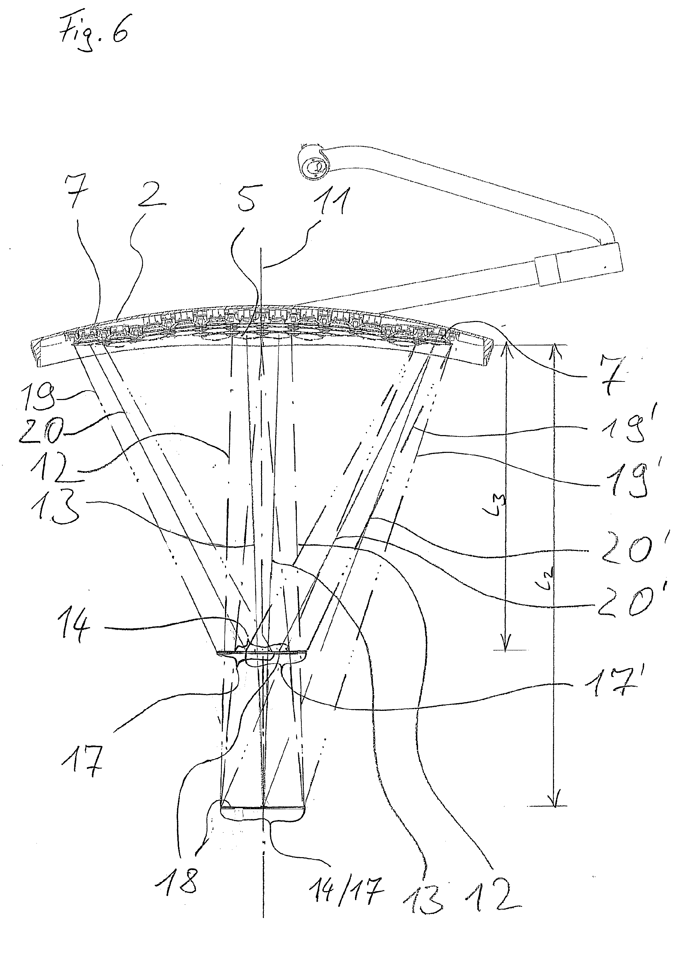

Fig. 6 zeigt eine weitere Ausführungsform der Operationsleuchte 1. In dieser Ausführungsform sind die ersten Lichtquellen 5, die unbeweglich in dem Leuchtenkörper 2 aufgenommen sind, und die dritten Lichtquellen 7, die kippbar in dem Leuchtenkörper 2 aufgenommen sind, vorgesehen. Die dritten Lichtquellen 7 sind um eine Kippachse kippbar, die im Wesentlichen tangential zu einem Kreis um die optische Achse 11 ist. Mehrere dritte Lichtquellen 7 sind dazu auf einem Halter befestigt, der mittels der Antriebsvorrichtung 10 um die Kippachse kippbar ist, oder alternativ einzeln kippbar zu dem Leuchtenkörper 2 befestigt und einzeln mittels jeweiliger Antriebsvorrichtungen 10 um die Kippachse kippbar. Die ersten Lichtquellen 5 sind ausschließlich, oder alternativ hauptsächlich, in dem in Fig. 2 gezeigten inneren Bereich I angeordnet. Die dritten Lichtquellen 7 sind ausschließlich, oder alternativ hauptsächlich, in dem in Fig. 2 gezeigten äußeren Bereich II angeordnet. Eine Gruppierung analog einer der zuvor beschriebenen Ausführungsformen ist auch hier alternativ möglich.

Wie in den vorangehenden Ausführungsformen werden durch die erste Lichtquelle 5 und die dritte Lichtquelle 7 auf der Operationsstelle jeweils ein erstes Leuchtfeld 14 und ein drittes Leuchtfeld 17 gebildet, die im Wesentlichen einen gleichen Durchmesser aufweisen, und eine gleiche Lichtverteilung haben. Auch hier sind aus Gründen der Übersichtlichkeit lediglich die Lichtbündel 12 mit jeweils der Achse 13 von zwei ersten Lichtquellen 5 und jeweils eines der Lichtbündel 19, 19' mit jeweils der Achse 20, 20' von der jeweiligen dritten Lichtquellen 7 auf den Abstand l₃ und eines der Lichtbündel 19' mit der Achse 20' auf den Abstand l₂ gezeigt.

Die weiteren Lichtquellen 5 bzw. 7 sind in dem Leuchtenkörper 2 so verteilt angeordnet, dass die Achsen ihrer Lichtbündel 12 bzw. 19 die optische Achse 11 in dem selben Punkt wie die Achse 13 bzw. 20 der gezeigten Lichtquellen 5 bzw. 7 schneiden, so dass sämtliche Lichtquellen 5 bzw. 7 das Leuchtfeld 14 bzw. 17, 17' bilden.

Auch in dieser Ausführungsform bilden die Leuchtfelder 14 und 17, 17' ein resultierendes Leuchtfeld 18. Gemäß der Anwendung muss dieses Leuchtfeld eine für Operationsleuchten normgerechte Lichtverteilung aufweisen. Dadurch ergibt sich eine voreingestellte relative Beleuchtungsstärke E_{cx} bei einem vorbestimmten Durchmesser dₓ.

Die Steuerungsvorrichtung 9 ist in dieser Ausführungsform so ausgebildet, dass sie die Lichtstärke der ersten Lichtquellen 5 und der dritten Lichtquellen 7 individuell ansteuern kann, sowie einen Kippwinkel der dritten Lichtquellen 7 ansteuern kann. Dadurch kann bei einem gewählten Abstand die voreingestellte relative Beleuchtungsstärke E_{cx} bei einem vorbestimmten Durchmesser dₓ erreicht werden.

Die Operationsleuchten 1 sind so ausgelegt, dass sie einen Arbeitsbereich haben, in dem die normativen Anforderungen eingehalten werden. Durch diesen Arbeitsbereich ergeben sich ein minimaler Arbeitsabstand und ein maximaler Arbeitsabstand zwischen dem Leuchtenkörper 2 und der Operationsstelle.

Um eine Vergrößerung bzw. eine Verkleinerung des vorbestimmten Durchmessers dₓ bei einer Abstandsänderung zu verhindern, steuert die Steuerungsvorrichtung 9 die Kippwinkel der dritten Lichtquellen 7 so, dass sich das dritte Leuchtfeld 17 radial zur optischen Achse 11 bewegt. Ferner wird dazu auch die Lichtstärke der ersten Lichtquellen 5 und dritten Lichtquellen 7 durch die Steuerungsvorrichtung 9 gesteuert. Ausgehend beispielsweise von dem resultierenden Lichtfeld 18 auf einem Körper in dem kleineren Abstand l₃ wird die dritte Lichtquelle 7 so verkippt, dass sich die Achse 20, 20' ihres Lichtbündels 19 von dem Schnittpunkt der optischen Achse 11 mit dem Körper im Abstand l₃ dann beispielsweise zu dem Schnittpunkt der optischen Achse 11 mit dem Körper im größeren Abstand l₂, also radial von der optischen Achse 11 weg bewegt. Dann wird das resultierende Lichtfeld 18 im Abstand l₃ gebildet. Die Lichtstärke der ersten Lichtquelle 5 und der dritten Lichtquelle 7 wird dann so angesteuert, dass der vorbestimmte Durchmesser dₓ im Wesentlichen unverändert bleibt. Analog erfolgt dieser Vorgang in umgekehrter Richtung.

Dies ist der prinzipielle Vorgang zum Konstanthalten. Tatsächlich ist es nicht unbedingt erforderlich, die Achsen sämtlicher Lichtquellen 5, 7 jeweils genau auf die Schnittpunkte zwischen der optischen Achse 11 und dem Körper in bestimmten Abständen zu richten. Um den Vorgang mit der Operationsleuchte 1 auszuführen, werden die Kippwinkel und die Beleuchtungsstärken E_{cx} sämtlicher Lichtquellen 5, 7 für die jeweiligen vorbestimmten Durchmesser dₓ bei verschiedenen Abständen empirisch ermittelt.

Um optional die zentrale Beleuchtungsstärke E_{c} konstant zu halten werden dann sowohl die Lichtstärken der einzelnen Lichtquellen 5, 7 als auch die Kippwinkel durch die Steuerungsvorrichtung 9 entsprechend angesteuert.

Um ein Konstanthalten des kleinsten vorbestimmten Durchmessers dx über den ganzen Arbeitsbereich zu ermöglichen, sind die Lichtquellen 5, 7 so ausgelegt und angeordnet, dass der vorbestimmte Durchmesser dₓ des resultierenden Leuchtfelds 18 in dem maximalen Arbeitsabstand maximal so groß, wie der kleinste vorbestimmbare Durchmesser dₓ ist. Das heißt, dass in einem Fall, in dem der vorbestimmte Durchmesser dₓ dem Leuchtfelddurchmesser d₁ entspricht, die Lichtquellen 5, 7 so ausgelegt und angeordnet sind, dass der Leuchtfelddurchmesser d₁ des resultierenden Leuchtfelds in dem maximalen Arbeitsabstand maximal so groß ist, wie der kleinste vorbestimmbare Leuchtfelddurchmesser d₁. Somit sind die Leuchtfelddurchmesser d₁ der einzelnen Leuchtfelder 14, 17 in dem maximalen Arbeitsabstand maximal so groß, wie der kleinste vorbestimmbare Leuchtfelddurchmesser d₁.

In einer alternativen Ausführungsform, können die Lichtquellen 5, 7 auch so ausgelegt und angeordnet sein, dass der vorbestimmte Durchmesser dₓ des resultierenden Leuchtfelds 18 in einem Abstand, der größer als der maximale Arbeitsabstand ist, maximal so groß, wie der kleinste vorbestimmbare Durchmesser dₓ ist. Auch dies bedeutet im Beispiel des Leuchtfelddurchmessers d1 als den vorbestimmten Durchmesser dₓ, dass der Leuchtfelddurchmesser d₁ der einzelnen Leuchtfelder in dem maximalen Arbeitsabstand maximal so groß ist, wie der kleinste vorbestimmbare Leuchtfelddurchmesser d₁.

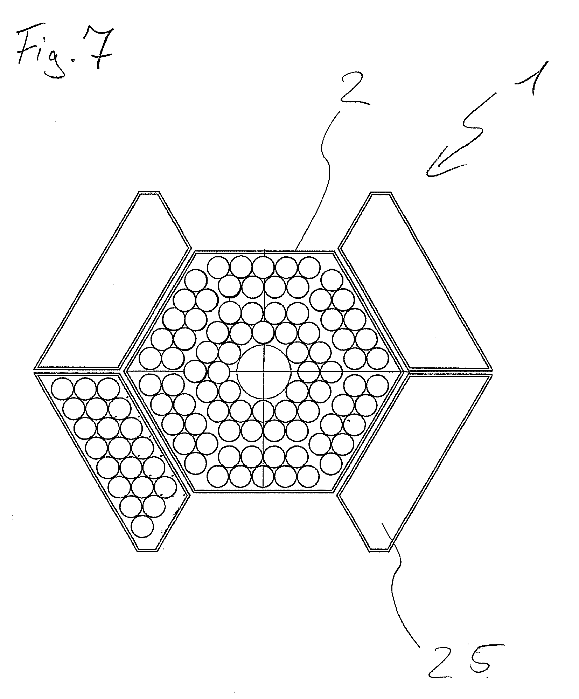

Fig. 7 zeigt eine weitere Ausführungsform der Operationsleuchte. Der Leuchenkörper 2 ist hier seitlich mit elektrischen und mechanischen Schnittstellen versehen. An diesen Schnittstellen sind Anbaumodule 25 anbringbar. Die Schnittstellen können alternativ als Standardschnittstellen, an die Anbaumodule 25 mit verschiedensten Funktionen anbringbar sind, vorgesehen sein. Die Anbaumodule 25 sind mit Lichtquellen 5 versehen, wobei die Lichtquellen 5 nur in einem Anbaumodul 25 dargestellt sind. In alternativen Ausführungsformen sind auch andere Lichtquellen 6, 7, 8 möglich. Weiterhin ist es auch möglich, alternativ weitere Lichtquellen, beispielsweise schmalbandige Lichtquellen für Fluoreszenzanregung, oder andere Bauteile, wie z.B. Sensoren oder eine Kamera in dem Anbaumodul vorzusehen.

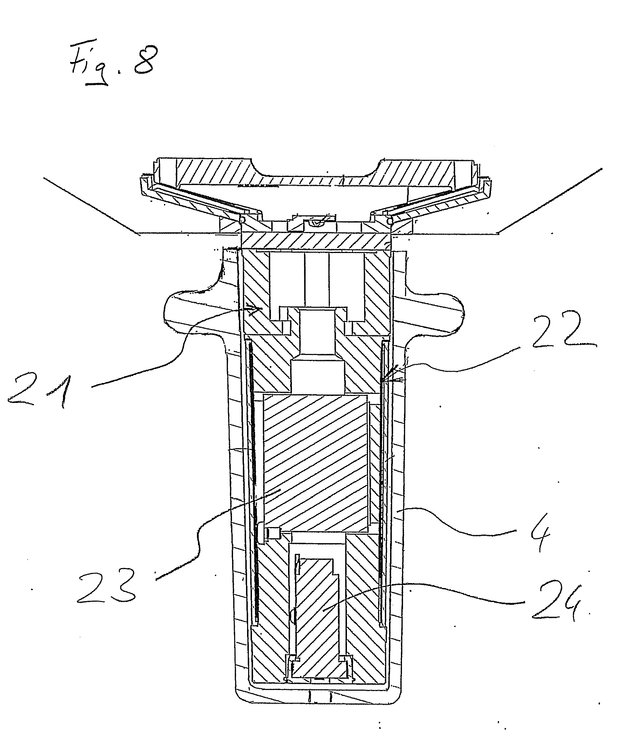

In Fig. 8 ist der Handgriff 4 der Operationsleuchte 1 gezeigt. Der Handgriff 4 ist über eine Handgriffaufnahme 21 geschoben und ist mit Hilfe eines nicht gezeigten Rastmechanismus befestigt. An der Handgriffaufnahme 21 ist eine Bedieneinrichtung mit zumindest einem Sensor 22 als Eingabemittel, und einer Auswerteeinheit 23 vorgesehen, die ein berührungsloses Bedienen der Operationsleuchte 1 ermöglicht.

Berührungslos heißt in diesem Zusammenhang, dass zwar die Bedieneinrichtung selbst, insbesondere der Sensor 22 der Bedieneinrichtung, nicht berührt wird, sterile Bauteile des Leuchtenkörpers, wie hier der Griff 4, die den Sensor der Bedieneinrichtung abdecken, jedoch berührt werden.

Mit der Bedieneinrichtung können der gewünschte Durchmesser dₓ und die gewünschte zentrale Beleuchtungsstärke E_{C} eingestellt werden. Das Einstellen erfolgt beispielsweise über ein Entlangfahren eines Objekts (z.B. der Finger des Operateurs) an dem Handgriff 4 in axialer Richtung für eine Einstellung beispielsweise des Durchmessers dₓ und Entlangfahren am Umfang des Handgriffs 4 für beispielsweise die Einstellung der zentralen Beleuchtungsstärke E_{C}.

Es können jedoch in anderen Ausführungsformen auch andere Einstellelemente, wie z.B. Druck- oder Drehschalter, Drehregler mit sterilem Bedienknopf, o.ä. vorgesehen sein, mit denen der gewünschte Durchmesser dₓ eingestellt werden kann oder alternativ voreingestellte Durchmesser dₓ ausgewählt werden können. Es werden hierbei die gewünschten Werte stufenlos eingestellt, oder alternativ aus vorher festgelegten Durchmessern dₓ oder vorher festgelegten zentralen Beleuchtungsstärken E_{C} ausgewählt.

In der Handgriffaufnahme 21 ist weiterhin eine Vorrichtung 24 zum Erfassen des Abstands zwischen dem Leuchtenkörper 2 und der Operationsstelle, hier in Form eines Abstandssensors, der als ein Lasersensor ausgebildet ist, vorgesehen. Mit dem Lasersensor wird der Abstand als Arbeitsabstand zwischen dem Leuchtenkörper 2 und der zu beleuchtenden Operationsstelle gemessen. Alternativ können auch andere Arten von Abstandsmessvorrichtungen, z.B. Ultraschallsensoren, oder z.B. Winkelaufnehmer im Tragsystem 3 zur Bestimmung der Position des Leuchtenkörpers 2, vorgesehen sein.

Die erforderlichen Werte zur Ansteuerung der Lichtstärke der einzelnen Lichtquellen 5, 6, 7, 8 und zur Ansteuerung der Antriebsvorrichtung 10 zum Einstellen des Kippwinkels für die dritten und vierten Lichtquellen 7, 8 werden in Abhängigkeit von dem gewünschten Durchmesser dₓ (Leuchtfelddurchmesser d₁₀), der zentralen Beleuchtungsstärke E_{C} der Operationsleuchte 1 und des Arbeitsabstands zwischen dem Leuchtenkörper 2 und dem zu beleuchtenden Objekt empirisch ermittelt und in einem Kennfeld in einem Speicherbereich der Steuerungsvorrichtung 9 gespeichert. Die Werte können dahingehend ermittelt werden, dass neben dem gewünschten Durchmesser dₓ (Leuchtfelddurchmesser d₁₀) auch das erforderliche Verhältnis von d₅₀ und d₁₀ eingehalten wird. Alternativ kann auch eine Beziehung der verschiedenen Werte hinterlegt werden.

Die Lichtquellen 5, 6, 7, 8 können zu Gruppen zusammengefasst sein, die jeweils über eine Ansteuereinheit 33 angesteuert werden. Das Kriterium für die Gruppierung kann der Leuchtfelddurchmesser d₁₀ des erzeugten Leuchtfelds 14, 17 oder der Abstand der Lichtquelle 5, 6, 7, 8 von der optischen Achse 11, also u.a. die Zugehörigkeit zum Bereich I oder zum Bereich II, sein, wobei auch innerhalb der Bereiche weitere Gruppierungen, z.B. Gruppierung nach der Farbtemperatur der LEDs, möglich sind. Die Lichtquellen 5, 6, 7, 8 der einzelnen Gruppen sind dann mit jeweils einer Ansteuereinheit 33 verbunden und können in ihrer Lichtstärke unterschiedlich angesteuert werden.

Im Betrieb misst der Lasersensor die Entfernung zwischen dem Leuchtenkörper 2 und dem zu beleuchtenden Körper und die Operationsleuchte 1 wird durch die Steuerungsvorrichtung 9 auf Sollanfangswerte für den Durchmesser dₓ, bei dem die voreingestellte relative Beleuchtungsstärke E_{cx} des resultierenden Leuchtfelds 18 vorliegt, und die zentrale Beleuchtungsstärke E_{C} zur Erzeugung eines resultierenden Leuchtfelds 18 eingestellt.

Die Sollvorgaben für den Durchmesser dₓ, bei dem die voreingestellte relative Beleuchtungsstärke E_{cx} des resultierenden Leuchtfelds 18 vorliegt, und die zentrale Beleuchtungsstärke E_{C} können mit Hilfe der Bedieneinrichtung verändert bzw. eingestellt werden.

Die Steuerung steuert dann die Lichtstärke der Lichtquellen 5, (6,) 7(, 8) und die Antriebsvorrichtung 10 der kippbaren Lichtquellen 7(, 8) so an, dass die gewünschte zentrale Beleuchtungsstärke E_{C} und der vorgegebene Durchmesser dₓ, bei dem die voreingestellte relative Beleuchtungsstärke E_{cx} des resultierenden Leuchtfelds 18 vorliegt, durch die Operationsleuchte 1 erzeugt werden. Entsprechend den Vorgaben für die zentrale Beleuchtungsstärke E_{C}, für den Durchmesser dₓ, bei dem die voreingestellte relative Beleuchtungsstärke E_{cx} des resultierenden Leuchtfelds 18 vorliegt, und dem gemessenen Abstand werden die Werte aus dem Speicherbereich abgerufen und als Betriebsdaten der Operationsleuchte 1 eingestellt.

Eine Positionsänderung des Leuchtenkörpers 2 oder eine Änderung von dessen Ausrichtung, also eine Veränderung des Arbeitsabstands, wird durch das Mittel 25 zum Auslösen der Änderung der individuellen Lichtstärken der Lichtquellen zum Anpassen des Leuchtfelds, hier den Bewegungssensor erfasst, und nach Beendigung der Bewegung wird der Abstand zwischen dem Leuchtenkörper 2 und dem zu beleuchtenden Körper durch den Lasersensor gemessen oder alternativ auf andere Art und Weise erfasst. Anhand dieses erfassten Werts werden der Durchmesser dₓ, bei dem die voreingestellte relative Beleuchtungsstärke E_{cx} des resultierenden Leuchtfelds 18 vorliegt, und die zentrale Beleuchtungsstärke E_{C} der Operationsleuchte 1 korrigiert, indem die nun zugehörigen Betriebsdaten aus dem Speicherbereich von der Steuerungsvorrichtung 9 abgerufen werden und die Lichtquellen 5, 6, 7, 8 in den einzelnen Gruppen durch die Steuerungsvorrichtung 9 mit einem entsprechenden Mischungsverhältnis der Bestromungsstärken angesteuert werden. Weiterhin wird die Antriebsvorrichtung 10 angesteuert, einen vorbestimmten Kippwinkel einzustellen. Durch diese Korrektur bleiben sowohl der Durchmesser dₓ des resultierenden Leuchtfelds 18 als auch, sofern entsprechend eingestellt, die zentrale Beleuchtungsstärke E_{C} auf dem Operationsfeld vor und nach der Bewegung unverändert.

Alternativ zu der Operationsleuchte 1, bei der sämtliche Lichtquellen 5, 6 in einem Leuchtenkörper 2 mit einem einzigen Gehäuse angeordnet sind, wobei optional Anbaumodule vorgesehen sein können, die nicht verkippbar sind, kann der Leuchtenkörper 2 auch mit mehreren Gehäusen, die als Module ausgebildet sind, vorgesehen sein, wobei dann jeweils eine Mehrzahl von starr angeordneten Lichtquellen 5, 6 vorgesehen ist, und die Lichtquellen 5, 6 miteinander in den verschiedenen Modulen angeordnet sind. Die Module können in dieser Ausführungsform zueinander kippbar sein, um die äußeren Lichtquellen so zu verkippen, dass sich ihre Achsen an gewünschten Punkten auf der optischen Achse des Leuchtenkörpers schneiden.

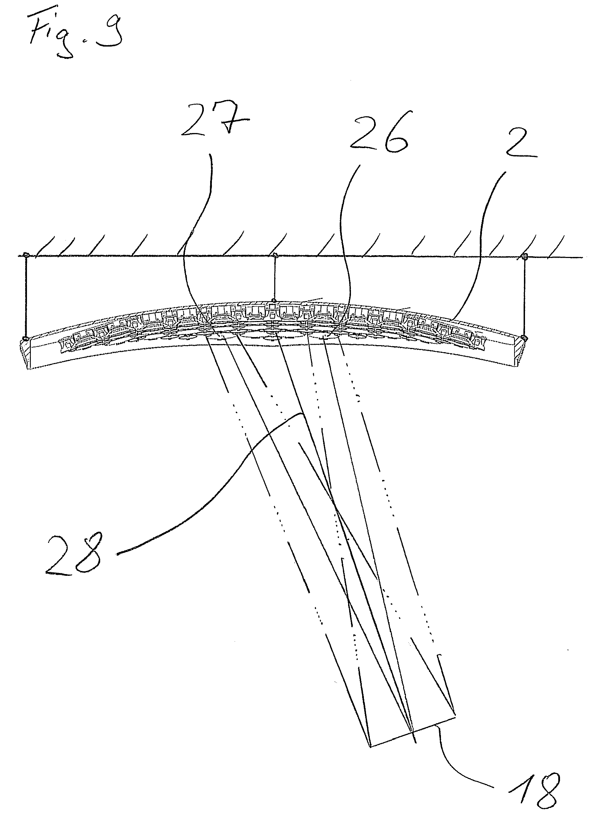

In einer weiteren Ausführungsform ist der Leuchtenkörper 2 der Operationsleuchte 1 nicht zwingend beweglich, sondern vorzugsweise starr vorgesehen. Beispielsweise ist der Leuchtenkörper 2, wie in Fig. 9 gezeigt, fest an einer Raumdecke befestigt. Alternativ ist die nachfolgend beschriebene Ausführungsform des Leuchtenkörpers auch für bewegliche Leuchtenkörper geeignet. In einer weiteren alternativen Ausführungsform sind die Lichtquellen und sonstige Bauteile direkt in der Raumdecke vorgesehen, die dann als Leuchtenkörper dient.

Eine optische Achse 28, auf der das resultierende Leuchtfeld 18 gebildet wird, und die in den vorangehenden Ausführungsformen dem Leuchtenkörper 2 fest zugeordnet ist, hat hier keinen festen Bezug zum Leuchtenkörper 2. Ein Winkel zwischen einer optischen Achse 28 und dem Leuchtenkörper 2 wird hier durch Lichtstrahlbündel von fünften Lichtquellen 26, die wiederum ein resultierendes Lichtstrahlbündel mit der schwenkbaren optischen Achse 28 bilden, definiert.

Die fünften Lichtquellen 26 sind daher, im Gegensatz zu den ersten Lichtquellen 5, die unbeweglich in dem Leuchtenkörper aufgenommen sind, um sämtliche Raumachsen schwenkbar in dem Leuchtenkörper 2 aufgenommen. Daher kann das resultierende Leuchtfeld 18 in einem festgelegten Bereich, beispielsweise der Fläche eines Operationstischs, eine beliebige Stelle beleuchten.

Ebenfalls besteht durch den Betrieb von beliebigen fünften Lichtquellen 26 die Möglichkeit, die Position der schwenkbaren optischen Achse 28 am Leuchtenkörper zu verändern.

Anstatt der dritten Lichtquellen 7 sind sechste Lichtquellen 27 vorgesehen, die ebenfalls um sämtliche Raumachsen schwenkbar sind. Die sechsten Lichtquellen 27 werden durch die Steuerungsvorrichtung 9 so angesteuert, dass sie bezüglich der schwenkbaren optischen Achse 28 prinzipiell die selben Kippbewegungen ausführen, wie die dritten Lichtquellen 7 bezüglich der optischen Achse 11.

Um bei einer nachträglichen Veränderung des vorher gewählten Abstands entlang der schwenkbaren optischen Achse 28, den vorbestimmten Durchmesser (dx) des resultierenden Leuchtfelds und optional die zentrale Beleuchtungsstärke konstant zu halten, ist eine Vorrichtung vorgesehen, die den Abstand zwischen dem Leuchtenkörper 2 und der Operationsstelle entlang der schwenkbaren optischen Achse 28 erfasst. Die Steuerungsvorrichtung 9 steuert dann auf der Basis der Abstandserfassung die Änderung des Kippwinkels der sechsten Lichtquellen 27 zu der schwenkbaren optischen Achse 28 prinzipiell analog zu der Änderung des Kippwinkels der dritten Lichtquellen 7 zu der optischen Achse 11 in den vorangehenden Ausführungsformen.

Im Folgenden sind Beispiele der Operationsleuchten und Verfahren, die nicht unter die Ansprüche fallen, beschrieben:

Erstes Beispiel der Operationsleuchte 1 zum Ausleuchten einer in einem gewählten Abstand angeordneten Operationsstelle, aufweisend: einen Leuchtenkörper 2 mit einer optischen Achse 11, der mindestens eine erste Lichtquelle 5 und eine zweite Lichtquelle 6 aufweist, wobei die erste Lichtquelle 5 ein erstes Leuchtfeld 14 und die zweite Lichtquelle 6 ein zweites Leuchtfeld 32 mit jeweils unterschiedlichen Durchmessern d1, d2 auf der Operationsstelle bilden, und die Leuchtfelder 14, 17 ein resultierendes im Wesentlichen kreisförmiges Leuchtfeld 18 mit einer Operationsleuchtennorm-gerechten Lichtverteilung (z.Zt. DIN EN 60601-2-41:2010) mit einer voreingestellten normgerechten relativen Beleuchtungsstärke E_{cx} bei einem vorbestimmten Durchmesser dₓ ergeben, eine Steuerungsvorrichtung 9 für die Lichtquellen 5, 6, die dazu angepasst ist, die Lichtstärke der ersten Lichtquelle 5 und die Lichtstärke der zweiten Lichtquelle 6 individuell anzusteuern, so dass bei dem gewählten Abstand die voreingestellte relative Beleuchtungsstärke E_{cx} bei dem vorbestimmten Durchmesser dₓ vorliegt, eine Vorrichtung 24 zum Erfassen des Abstands zwischen dem Leuchtenkörper 2 und der Operationsstelle, wobei bei einer nachträglichen Veränderung des vorher gewählten Abstands zwischen dem Leuchtenkörper 2 und der Operationsstelle die Steuerungsvorrichtung 9 dazu angepasst ist, die Lichtstärken der einzelnen Lichtquellen 5, 6 individuell so anzusteuern, dass der Durchmesser dₓ auf der Operationsstelle, bei dem die voreingestellte relative Beleuchtungsstärke E_{cx} des resultierenden Leuchtfelds 18 vorliegt, im Wesentlichen unverändert bleibt.

Zweites Beispiel der Operationsleuchte 1 entsprechend der Operationsleuchte 1 gemäß dem ersten Beispiel, mit einem Mittel 25 zum Auslösen der Änderung der individuellen Ansteuerung der Lichtstärken der Lichtquellen 5, 6.

Drittes Beispiel der Operationsleuchte 1 entsprechend der Operationsleuchte 1 gemäß dem ersten oder zweiten Beispiel, wobei die Steuerungsvorrichtung 9 dazu angepasst ist, dass bei einer Veränderung des Abstands eine normgerechte zentrale Beleuchtungsstärke E_{c} des resultierenden Leuchtfelds 18 unverändert bleibt.

Viertes Beispiel der Operationsleuchte 1 entsprechend der Operationsleuchte 1 gemäß einem vom dem ersten bis dritten Beispiel, wobei zumindest ein Teil der Lichtquellen 5, 6 verkippbar in dem Leuchtenkörper 2 angeordnet sind.

Fünftes Beispiel der Operationsleuchte 1 entsprechend der Operationsleuchte 1 gemäß einem vom dem ersten bis dritten Beispiel, wobei die Operationsleuchte 1 mehrere Module aufweist, in denen jeweils mindestens eine der ersten Lichtquellen 5 und mindestens eine der zweiten Lichtquellen 6 angeordnet sind, wobei die Module zueinander kippbar sind.

Sechstes Beispiel der Operationsleuchte 1 entsprechend der Operationsleuchte 1 gemäß einem vom dem ersten bis dritten Beispiel, wobei eine Lichtaustrittsfläche 29 des Leuchtenkörpers 2 in einen im Wesentlichen kreisförmigen inneren Bereich I und mindestens einen darum herum angeordneten äußeren Bereich II unterteilt ist, wobei die ersten und zweiten Lichtquellen 5, 6 starr angeordnet zumindest in dem inneren Bereich I vorhanden sind, und mindestens eine dritte Lichtquelle 7 und mindestens eine vierte Lichtquelle 8, die jeweils ein Leuchtfeld mit unterschiedlichen Durchmessern bilden, in dem mindestens einen äußeren Bereich II vorgesehen sind, und die dritten und vierten Lichtquellen 7, 8 in dem Leuchtenkörper 2 verkippbar gelagert sind und eine über die Steuerungsvorrichtung 9 gesteuerte Antriebsvorrichtung 10 zum Kippen der dritten und vierten Lichtquellen 7, 8 um einen Kippwinkel aufweisen, wobei die Steuerungsvorrichtung 9 dazu angepasst ist, die Lichtstärken der dritten Lichtquellen 7 und der vierten Lichtquellen 8 und die Antriebsvorrichtung 10 so anzusteuern, dass der Durchmesser dₓ, bei dem die voreingestellte relative Beleuchtungsstärke E_{cx} des resultierenden Leuchtfelds 18 vorliegt, im Wesentlichen unverändert bleibt.

Siebtes Beispiel der Operationsleuchte 1 entsprechend der Operationsleuchte 1 gemäß dem zweiten Beispiel und einem vom dem dritten bis sechsten Beispiel, wobei das Mittel 25 zum Auslösen der Änderung der individuellen Ansteuerung der Lichtstärken ein Bewegungssensor ist, und die Steuerungsvorrichtung 9 so ausgebildet ist, dass sie nach einer von dem Bewegungssensor erfassten abgeschlossenen Bewegung des Leuchtenkörpers 2 den erfassten Abstand zwischen dem Leuchtenkörper 2 und der Operationsstelle auswertet und die Lichtquellen 5, 6, 7, 8 entsprechend ansteuert.

Achtes Beispiel der Operationsleuchte 1 entsprechend der Operationsleuchte 1 gemäß einem der vorangehenden Beispiele, wobei die erste Lichtquelle 5 eine erste Linse aufweist und die zweite Lichtquelle 6 eine zweite Linse aufweisen, und die erste Linse und die zweite Linse jeweils unterschiedliche optisch wirksame Flächen aufweisen, die so angepasst sind, dass die erzeugten Lichtfelder unterschiedliche Lichtverteilungen aufweisen.

Neuntes Beispiel der Operationsleuchte 1 entsprechend der Operationsleuchte 1 gemäß einem der vorangehenden Beispiele, wobei die erste Lichtquelle 5 eine erste Linse aufweist und die zweite Lichtquelle 6 eine zweite Linse aufweisen, und die erste Linse und die zweite Linse jeweils unterschiedliche Durchmesser aufweisen.

Zehntes Beispiel der Operationsleuchte 1 entsprechend der Operationsleuchte 1 gemäß einem der vorangehenden Beispiele wobei die Operationsleuchte 1 mindestens ein mit der Steuerungsvorrichtung 9 verbundenes Eingabemittel 22 zum Einstellen des Durchmessers dₓ, bei dem die voreingestellte relative Beleuchtungsstärke E_{cx} des resultierenden Leuchtfelds 18 vorliegt, aufweist.

Elftes Beispiel der Operationsleuchte 1 entsprechend der Operationsleuchte 1 gemäß dem zehnten Beispiel, wobei das Eingabemittel 22 ein Mittel zum Auswählen aus verschiedenen auswählbar voreingestellten Durchmessern dₓ, bei denen die voreingestellte relative Beleuchtungsstärke E_{cx} vorliegt, ist.

Zwölftes Beispiel der Operationsleuchte 1 entsprechend der Operationsleuchte 1 gemäß einem der vorangehenden Beispiele, wobei die Lichtquellen 5, 6, 7, 8 in Gruppen zusammengefasst sind, wobei zumindest ein Kriterium für die Gruppierung der Durchmesser d1, d2 des durch die Lichtquellen 5, 6, 7, 8 erzeugten Leuchtfelds 14, 17 ist und ein weiteres Kriterium der Abstand der Lichtquelle 5, 6, 7, 8 von der optischen Achse 11 ist, und die Steuerungsvorrichtung 9 so angepasst ist, dass die Lichtquellen 5, 6, 7, 8 in den einzelnen Gruppen jeweils gleich, und die Gruppen individuell angesteuert werden können.

Dreizehntes Beispiel der Operationsleuchte 1 entsprechend der Operationsleuchte 1 gemäß dem sechsten Beispiel und einem von dem siebten bis zwölften Beispiel, wobei die dritten und vierten Lichtquellen 7, 8 im äußersten Bereich II angeordnet sind. Vierzehntes Beispiel der Operationsleuchte 1 entsprechend der Operationsleuchte 1 gemäß dem zwölften oder dreizehnten Beispiel, wobei die Steuerungsvorrichtung 9 einen Speicherbereich aufweist, und die Steuerungsvorrichtung 9 so ausgebildet ist, dass die Lichtstärken der Lichtquellen 5, 6, 7, 8 in den einzelnen Gruppen als Bestromungsstärke in Form eines Kennfelds in dem Speicherbereich hinterlegt sind und abhängig von dem Abstand zwischen dem Leuchtenkörper 2 und der Operationsstelle als Mischungsverhältnis von der Steuerungsvorrichtung abrufbar sind.

Fünfzehntes Beispiel der Operationsleuchte 1 entsprechend der Operationsleuchte 1 gemäß dem sechsten Beispiel und dem vierzehnten Beispiel, wobei gemäß die Steuerungsvorrichtung 9 so ausgebildet ist, dass der Kippwinkel in dem Speicherbereich in Abhängigkeit von dem Abstand zwischen dem Leuchtenkörper 2 und der Operationsstelle gespeichert ist, und von der Steuerungsvorrichtung 9 entsprechend dem Abstand zwischen dem Leuchtenkörper 2 und der Operationsstelle abrufbar ist.

Erstes beispielhaftes Verfahren zum Betreiben einer Operationsleuchte 1 gemäß einem der vorangehenden Beispiele, mit den folgenden Schritten:
Erfassen einer Änderung eines Abstands zwischen dem Leuchtenkörper 2 und der Operationsstelle,
Ändern der jeweiligen Lichtstärke der mindestens ersten Lichtquelle 5 und der mindestens zweiten Lichtquelle 6, so dass der vor der Änderung des Abstandes eingestellte Durchmesser dₓ, bei dem die voreingestellte relative Beleuchtungsstärke E_{cx} des resultierenden Leuchtfelds 18 vorliegt, im Wesentlichen unverändert bleibt.
Zweites beispielhaftes Verfahren entsprechend dem ersten beispielhaften Verfahren,
wobei der gewünschte Durchmesser dₓ, bei dem die voreingestellte relative Beleuchtungsstärke E_{cx} des resultierenden Leuchtfelds 18 vorliegt, vorab eingestellt wird.

Drittes beispielhaftes Verfahren entsprechend dem ersten oder zweiten beispielhaften Verfahren,
wobei die gewünschte zentrale Beleuchtungsstärke E_{c} des resultierenden Leuchtfelds 18 vorab eingestellt wird.

Viertes beispielhaftes Verfahren entsprechend einem von dem ersten bis dritten beispielhaften Verfahren,
wobei nach dem Beendigen einer Veränderung des Abstands das Anpassen des Durchmessers dₓ, bei dem die voreingestellte relative Beleuchtungsstärke E_{cx} des resultierenden Leuchtfelds 18 vorliegt, ausgelöst wird.

Fünftes beispielhaftes Verfahren entsprechend einem von dem ersten bis vierten beispielhaften Verfahren,
wobei für ein Verhindern einer Vergrößerung des Durchmessers dₓ, bei dem die voreingestellte relative Beleuchtungsstärke E_{cx} des resultierenden Leuchtfelds 18 vorliegt, die erste Lichtquelle 5, die das Leuchtfeld 14 mit dem kleineren Durchmesser d1 erzeugt, so angesteuert wird, dass die Lichtstärke der ersten Lichtquelle 5 erhöht wird und/oder die zweite Lichtquelle 6, die das Leuchtfeld 17 mit dem größeren Durchmesser d2 erzeugt, so angesteuert wird, dass die Lichtstärke der zweiten Lichtquelle 6 verringert wird, und
wobei für ein Verhindern einer Verkleinerung des Durchmessers dₓ, bei dem die voreingestellte relative Beleuchtungsstärke E_{cx} des resultierenden Leuchtfelds 18 vorliegt, die erste Lichtquelle 5, die das Leuchtfeld 14 mit dem kleineren Durchmesser d1 erzeugt, so angesteuert wird, dass die Lichtstärke der ersten Lichtquelle 5 verringert wird und/oder die zweite Lichtquelle 6, die das Leuchtfeld 17 erzeugt, so angesteuert wird, dass die Lichtstärke der zweiten Lichtquelle 6 erhöht wird.

## Patentansprüche

1. Operationsleuchte (1) zum Ausleuchten einer in einem gewählten Abstand entlang einer optischen Achse (11) angeordneten Operationsstelle, aufweisend:
einen Leuchtenkörper (2) mit der optischen Achse (11), der mindestens eine erste Lichtquelle (5) und eine dritte Lichtquelle (7) aufweist, wobei
die erste Lichtquelle (5) unbeweglich in dem Leuchtenkörper (2) aufgenommen ist und die dritte Lichtquelle (7) kippbar in dem Leuchtenkörper (2) aufgenommen ist,
die erste Lichtquelle (5) ein erstes Leuchtfeld (14) und die dritte Lichtquelle (7) ein drittes Leuchtfeld (17, 17') mit jeweils im Wesentlichen gleichen Durchmessern (d1) und im Wesentlichen gleichen Lichtverteilungen auf der Operationsstelle bilden, und
die Leuchtfelder (14, 17, 17' ) ein resultierendes, im Wesentlichen kreisförmiges Leuchtfeld (18) mit einer Operationsleuchtennorm-gerechten Lichtverteilung (z.Zt. DIN EN 60601-2-41:2010) mit einer voreingestellten normgerechten relativen Beleuchtungsstärke (E_{cx}) bei einem vorbestimmten Durchmesser (dₓ) auf der optischen Achse (11) ergeben;
eine Steuerungsvorrichtung (9) für die Lichtquellen (5, 7), die dazu angepasst ist, die Lichtstärke der ersten Lichtquelle (5) und der dritten Lichtquelle (7) individuell anzusteuern, sowie einen Kippwinkel der dritten Lichtquelle (7) anzusteuern, so dass bei dem gewählten Abstand die voreingestellte relative Beleuchtungsstärke (E_{cx}) bei dem vorbestimmten Durchmesser (dₓ) vorliegt;
Ansteuereinheiten (33), über die die Lichtquellen (5, 7) jeweils mit der Steuerungsvorrichtung (9) verbunden sind,
eine Antriebsvorrichtung (10) zum Kippen der dritten Lichtquelle (7), die mit der Steuerungsvorrichtung (9) verbunden ist,
eine Vorrichtung (24) zum Erfassen eines Abstands zwischen dem Leuchtenkörper (2) und der Operationsstelle entlang der optischen Achse (11), und
ein Mittel (25) zum Auslösen der Änderung der individuellen Ansteuerung der Lichtstärken der Lichtquellen (5, 7) bzw. der Änderung des Kippwinkels der dritten Lichtquelle (7),
**dadurch gekennzeichnet, dass**
bei einer nachträglichen Veränderung des vorher gewählten Abstands zwischen dem Leuchtenkörper (2) und der Operationsstelle die Steuerungsvorrichtung (9) dazu angepasst ist, durch das Mittel (25) ausgelöst, die Lichtstärken der einzelnen Lichtquellen (5, 7) individuell anzusteuern sowie den Kippwinkel der dritten Lichtquelle (7) anzusteuern, so dass der vorbestimmte Durchmesser (dₓ) auf der Operationsstelle, bei dem die voreingestellte relative Beleuchtungsstärke (E_{cx}) des resultierenden Leuchtfelds (10) vorliegt, im Wesentlichen unverändert bleibt.

2. Operationsleuchte (1) gemäß Anspruch 1, **dadurch gekennzeichnet, dass** die Operationsleuchte (1) mehrere Module aufweist, wobei in einem inneren Modul mindestens eine der ersten Lichtquellen (5) und mindestens eine der dritten Lichtquellen (7) angeordnet sind, und in den weiteren Modulen dritte Lichtquellen (7) angeordnet sind.

3. Operationsleuchte (1) gemäß Anspruch 1 oder 2, **dadurch gekennzeichnet, dass**
eine Lichtaustrittsfläche (29) des Leuchtenkörpers (2) bzw. des inneren Moduls in einen im Wesentlichen kreisförmigen inneren Bereich (I) und mindestens einen darum herum angeordneten äußeren Bereich (II) unterteilt ist,
wobei die mindestens eine erste Lichtquelle (5) zumindest in dem inneren Bereich (I) vorhanden ist, und
die mindestens eine dritte Lichtquelle (7) zumindest in dem mindestens einen äußeren Bereich (II) vorgesehen ist.

4. Operationsleuchte (1) gemäß einem der Ansprüche 1 bis 3, **dadurch gekennzeichnet, dass** die Lichtquellen (5, 7) in Gruppen zusammengefasst sind, wobei zumindest ein Kriterium für die Gruppierung das unbewegliche oder kippbare Aufnehmen der Lichtquellen (5, 7) ist, und die Steuerungsvorrichtung (9) so angepasst ist, dass die Lichtquellen (5, 7) in den einzelnen Gruppen jeweils gleich, und die Gruppen individuell angesteuert werden können.

5. Operationsleuchte (1) gemäß Anspruch 4, **dadurch gekennzeichnet, dass** die Steuerungsvorrichtung (9) einen Speicherbereich aufweist, und die Steuerungsvorrichtung (9) so ausgebildet ist, dass die Lichtstärken der Lichtquellen (5, 7) in den einzelnen Gruppen als Bestromungsstärke in Form eines Kennfelds und der Kippwinkel in dem Speicherbereich hinterlegt sind und abhängig von dem Abstand zwischen dem Leuchtenkörper (2) und der Operationsstelle als Mischungsverhältnis von der Steuerungsvorrichtung (9) abrufbar sind.

6. Operationsleuchte (1) zum Ausleuchten einer in einem gewählten Abstand entlang einer schwenkbaren optischen Achse (28) angeordneten Operationsstelle, aufweisend:
einen Leuchtenkörper (2) mit der schwenkbaren optischen Achse (28), der mehrere fünfte Lichtquellen (26), und mindestens eine sechste Lichtquelle (27) aufweist, wobei
die fünften Lichtquellen (26) schwenkbar in dem Leuchtenkörper (2) aufgenommen sind und mehrere der fünften Lichtquellen (26) ein resultierendes Lichtbündel mit der schwenkbaren optischen Achse (28) bilden, und die mindestens eine sechste Lichtquelle (27) schwenkbar in dem Leuchtenkörper (2) aufgenommen ist,
die fünften Lichtquellen (26) ein erstes Leuchtfeld und die mindestens eine sechste Lichtquelle (27) ein drittes Leuchtfeld mit jeweils im Wesentlichen gleichen Durchmessern (d1) und im Wesentlichen gleichen Lichtverteilungen auf der Operationsstelle bilden, und
die Leuchtfelder ein resultierendes, im Wesentlichen kreisförmiges Leuchtfeld (18) mit einer Operationsleuchtennorm-gerechten Lichtverteilung (z.Zt. DIN EN 60601-2-41:2010) mit einer voreingestellten normgerechten relativen Beleuchtungsstärke (E_{cx}) bei einem vorbestimmten Durchmesser (dₓ) auf der schwenkbaren optischen Achse (28), auf der das Leuchtfeld (18) gebildet ist, ergeben;
eine Steuerungsvorrichtung (9) für die Lichtquellen (26, 27), die dazu angepasst ist, die Lichtstärke der fünften Lichtquellen (5) und der mindestens einen sechsten Lichtquelle (27) individuell anzusteuern, sowie einen Kippwinkel der mindestens einen sechsten Lichtquelle (27) anzusteuern, so dass bei dem gewählten Abstand die voreingestellte relative Beleuchtungsstärke (E_{cx}) bei dem vorbestimmten Durchmesser (dₓ) vorliegt;
Ansteuereinheiten (33), über die die Lichtquellen (27, 27) jeweils mit der Steuerungsvorrichtung (9) verbunden sind,
eine Antriebsvorrichtung (10) zum Kippen der mindestens einen sechsten Lichtquelle (7), die mit der Steuerungsvorrichtung (9) verbunden ist,
eine Vorrichtung (24) zum Erfassen eines Abstands zwischen dem Leuchtenkörper (2) und der Operationsstelle entlang der schwenkbaren optischen Achse (28), und
ein Mittel (25) zum Auslösen der Änderung der individuellen Ansteuerung der Lichtstärken der Lichtquellen (26, 27) bzw. der Änderung des Kippwinkels der mindestens einen sechsten Lichtquelle (27),
**dadurch gekennzeichnet, dass**
bei einer nachträglichen Veränderung des vorher gewählten Abstands zwischen dem Leuchtenkörper (2) und der Operationsstelle die Steuerungsvorrichtung (9) dazu angepasst ist, durch das Mittel (25) ausgelöst, die Lichtstärken der einzelnen Lichtquellen (26, 27) individuell anzusteuern sowie den Kippwinkel der mindestens einen sechsten Lichtquelle (7) anzusteuern, so dass der vorbestimmte Durchmesser (dₓ) auf der Operationsstelle, bei dem die voreingestellte relative Beleuchtungsstärke (E_{cx}) des resultierenden Leuchtfelds (18) vorliegt, im Wesentlichen unverändert bleibt.

7. Operationsleuchte (1) gemäß einem der Ansprüche 1 bis 6, wobei der vorbestimmte Durchmesser (dₓ) des resultierenden Leuchtfelds (18) in einem Abstand, der gleich einem maximalen Arbeitsabstand ist, maximal so groß ist, wie der kleinste vorbestimmbare Durchmesser (dₓ).

8. Operationsleuchte (1) gemäß einem der Ansprüche 1 bis 6, wobei der vorbestimmte Durchmesser (dₓ) des resultierenden Leuchtfelds (18) in einem Abstand, der größer als ein maximaler Arbeitsabstand ist, maximal so groß ist, wie der kleinste vorbestimmbare Durchmesser (dₓ).

9. Operationsleuchte (1) gemäß einem der Ansprüche 1 bis 8, **dadurch gekennzeichnet, dass** die Steuerungsvorrichtung (9) dazu angepasst ist, dass bei einer Veränderung des Abstands eine normgerechte zentrale Beleuchtungsstärke (E_{c}) des resultierenden Leuchtfelds (18) unverändert bleibt.

10. Operationsleuchte (1) gemäß einem der Ansprüche 1 bis 9, **dadurch gekennzeichnet, dass** das Mittel (25) zum Auslösen der Änderung der individuellen Ansteuerung der Lichtstärken bzw. der Änderung des Kippwinkels ein Bewegungssensor ist, und die Steuerungsvorrichtung (9) so ausgebildet ist, dass sie nach einer von dem Bewegungssensor erfassten abgeschlossenen Bewegung des Leuchtenkörpers (2) den erfassten Abstand zwischen dem Leuchtenkörper (2) und der Operationsstelle auswertet und die Lichtquellen (5, 7) entsprechend ansteuert.

11. Operationsleuchte (1) gemäß einem der Ansprüche 1 bis 10, **dadurch gekennzeichnet, dass** die Operationsleuchte (1) mindestens ein mit der Steuerungsvorrichtung (9) verbundenes Eingabemittel (22) zum Einstellen des Durchmessers (dₓ), bei dem die voreingestellte relative Beleuchtungsstärke (E_{cx}) des resultierenden Leuchtfelds (18) vorliegt, aufweist.

12. Operationsleuchte (1) gemäß Anspruch 11, **dadurch gekennzeichnet, dass** das Eingabemittel (22) ein Mittel zum Auswählen aus verschiedenen auswählbar voreingestellten Durchmessern (dₓ) ist, bei denen die voreingestellte relative Beleuchtungsstärke (E_{cx}) vorliegt.

13. Verfahren zum Betreiben einer Operationsleuchte (1) gemäß einem der Ansprüche 1 bis 12, mit den folgenden Schritten:
Erfassen einer Änderung eines Abstands zwischen dem Leuchtenkörper (2) und der Operationsstelle,
Ändern der jeweiligen Lichtstärke der mindestens ersten Lichtquelle (5) bzw. fünften Lichtquelle (26) und der mindestens dritten Lichtquelle (7) bzw. sechsten Lichtquelle (27), und Ändern des Kippwinkels der mindestens dritten Lichtquelle (7) bzw. sechsten Lichtquelle (27), so dass der vor der Änderung des Abstandes eingestellte Durchmesser (dₓ), im Wesentlichen unverändert bleibt.

14. Verfahren gemäß Anspruch 13,
wobei der gewünschte Durchmesser (dₓ), bei dem die voreingestellte relative Beleuchtungsstärke (E_{cx}) des resultierenden Leuchtfelds (18) vorliegt, vorab eingestellt wird.

15. Verfahren gemäß einem der Ansprüche 13 oder 14,
wobei die gewünschte zentrale Beleuchtungsstärke (E_{c}) des resultierenden Leuchtfelds (18) vorab eingestellt wird.

16. Verfahren gemäß einem der Ansprüche 13 bis 15,
wobei nach dem Beendigen einer Veränderung des Abstands das Anpassen des Durchmessers (dₓ), bei dem die voreingestellte relative Beleuchtungsstärke (E_{cx}) des resultierenden Leuchtfelds (18) vorliegt, ausgelöst wird.

17. Verfahren gemäß einem der Ansprüche 14 bis 16,
wobei für ein Verhindern einer Vergrößerung des Durchmessers (dₓ), bei dem die voreingestellte relative Beleuchtungsstärke (E_{cx}) des resultierenden Leuchtfelds (18) vorliegt, der Kippwinkel der dritten Lichtquelle (7) bzw. der sechsten Lichtquelle (27) so angesteuert wird, dass sich das dritte Leuchtfeld (17, 17') radial in Richtung zu der optischen Achse (11) bzw. zu der schwenkbaren optischen Achse (28) hin bewegt, und die erste Lichtquelle (5) bzw. fünfte Lichtquelle (26), die das erste Leuchtfeld (14) erzeugt, und die dritte Lichtquelle (7) bzw. die sechste Lichtquelle (27), die das dritte Leuchtfeld (17, 17') erzeugt, so angesteuert werden, dass der Durchmessers (dₓ) mit der relativen Beleuchtungsstärke (E_{cx}) im Wesentlichen unverändert bleibt, und
wobei für ein Verhindern einer Verkleinerung des Durchmessers (dₓ), bei dem die voreingestellte relative Beleuchtungsstärke (E_{cx}) des resultierenden Leuchtfelds (18) vorliegt, der Kippwinkel der dritten Lichtquelle (7) bzw. der sechsten Lichtquelle (27) so angesteuert wird, dass sich das dritte Leuchtfeld (17, 17') radial in Richtung von der optischen
Achse (11) bzw. von der schwenkbaren optischen Achse (28) weg bewegt, und die erste Lichtquelle (5) bzw. die fünfte Lichtquelle (26) und die dritte Lichtquelle (7) bzw. die sechste Lichtquelle (27) so angesteuert werden, dass der Durchmesser (dₓ) mit der relativen Beleuchtungsstärke (E_{cx}) im Wesentlichen unverändert bleibt.

18. Verfahren gemäß Anspruch 17, mit einer Operationsleuchte gemäß Anspruch 9 oder Anspruch 9 und einem der Ansprüche 10 bis 12,
wobei bei dem Verhindern der Vergrößerung des Durchmessers (dₓ), bei dem die voreingestellte relative Beleuchtungsstärke (E_{cx}) des resultierenden Leuchtfelds (18) vorliegt, der Kippwinkel der dritten Lichtquelle (7) bzw. der sechsten Lichtquelle (27) so angesteuert wird, dass sich das dritte Leuchtfeld (17, 17') radial in Richtung zu der optischen Achse (11) bzw. der schwenkbaren optischen Achse (28) bewegt, und die erste Lichtquelle (5) bzw. die fünfte Lichtquelle (26), die das erste Leuchtfeld (14) erzeugt, und die dritte Lichtquelle (7) bzw. die sechste Lichtquelle (27), die das dritte Leuchtfeld (17, 17') erzeugt, so angesteuert werden, dass auch die zentrale Beleuchtungsstärke (E_{c}) im Wesentlichen unverändert bleibt, und
wobei bei dem Verhindern der Verkleinerung des Durchmessers (dₓ), bei dem die voreingestellte relative Beleuchtungsstärke (E_{cx}) des resultierenden Leuchtfelds (18) vorliegt, der Kippwinkel der dritten Lichtquelle (7) bzw. der sechsten Lichtquelle (27) so angesteuert wird, dass sich das dritte Leuchtfeld (17, 17') radial in Richtung von der optischen Achse (11) bzw. von der schwenkbaren optischen Achse (28) weg bewegt, und die erste Lichtquelle (5) bzw. die fünfte Lichtquelle (26) und die dritte Lichtquelle (7) bzw. die sechste Lichtquelle (27) so angesteuert werden, dass auch die zentrale Beleuchtungsstärke (E_{c}) im Wesentlichen unverändert bleibt.

## Claims

1. Surgical lamp (1) for illuminating an operating site located at a chosen distance along an optical axis (11) comprising:
a lamp body (2), having the optical axis (11), comprising at least one first light source (5) and one third light source (7), wherein
the first light source (5) is immovably accommodated in the lamp body (2) and the third light source (7) is accommodated in a tiltable manner in the lamp body (2),
the first light source (5) generates a first light field (14) and the third light source (7) generates a third light field (17, 17') respectively having substantially identical diameters (d1) and substantially identical light distributions on the operating site, and
the light fields (14, 17, 17') yield, along the optical axis (11), a resultant light field (18) that has a substantially circular shape and is associated with a light distribution that conforms to a surgical lamp standard (at the moment: DIN EN 60601-2-41:2010) and that has a preset relative illuminance (E_{cx}) conforming to that standard at a predetermined diameter (dₓ);
a control device (9) for the light sources (5, 7) configured to individually control the light intensity of the first light source (5) and of the third light source (7) and to control a tilting angle of the third light source (7) such that the preset relative illuminance (E_{cx}) is provided at the predetermined diameter (dₓ) at the chosen distance;
actuating units (33), by means of which, the light sources (5, 7) are respectively connected to the control device (9),
a driving device (10) connected to the control device (9) for tilting the third light source (7),
a device (24) for detecting a distance between the lamp body (2) and the operating site along the optical axis (11), and
a means (25) for triggering the change of the individual control of the light intensities of the light sources (5,7) or the change of the tilting angle of the third light source (7),
**characterized in that**,
when the previously chosen distance between the lamp body (2) and the operating site is subsequently changed, the control device (9) is configured, triggered by the means (25), to individually control the light intensities of the individual light sources (5, 7) and the tilting angle of the third light source (7) such that the predetermined diameter (dₓ) where the preset relative illuminance (E_{cx}) of the resultant light field (10) exists on the operating site is maintained at a substantially constant value.

2. Surgical lamp (1) according to claim 1, **characterized in that** the surgical lamp (1) comprises several modules, wherein, in an inner module, at least one of the first light sources (5) and at least one of the third light sources (7) are arranged and, in the further modules, third light sources (7) are arranged.

3. Surgical lamp (1) according to claim 1 or 2, **characterized in that**
a light-emitting surface (29) of the lamp body (2) or of the inner module is divided into an inner area (I) that has a substantially circular shape and at least one outer area (II) positioned around the inner area (I),
wherein the at least one first light source (5) is at least provided in the inner area (I), and
the at least one third light source (7) is at least provided in the at least one outer area (II).

4. Surgical lamp (1) according to any of the claims 1 to 3, **characterized in that** the light sources (5, 7) are assigned to groups, wherein at least one criteria for the assignment is the immovable or tiltable accommodation of the light sources (5, 7), and the control device (9) is configured such that the light sources (5, 7) within the individual groups can respectively be controlled similarly and the groups can be controlled independently of one another.

5. Surgical lamp (1) according to claim 4, **characterized in that** the control device (9) comprises a storage area, and the control device (9) is configured such that the light intensities of the light sources (5, 7) in the individual groups are stored as force values associated with currents in a mapping and the tilting angle is stored in the storage area and that they are retrievable by the control device (9) as a mixing ratio that depends on the distance between the lamp body (2) and the operating site.

6. Surgical lamp (1) for illuminating an operating site located at a chosen distance along a tiltable optical axis (28) comprising:
a lamp body (2), having the tiltable optical axis (28), comprising several fifth light sources (26) and at least one sixth light source (27), wherein
the fifth light sources (26) are accommodated in a tiltable manner in the lamp body (2) and several of the fifth light sources (26) generate a resultant light sheath having the tiltable optical axis (28), and the at least one sixth light source (27) is accommodated in the lamp body (2) in a tiltable manner,
the fifth light sources (26) generate a first light field and the at least one sixth light source (27) generates a third light field respectively having diameters (d₁) that are substantially equal and substantially equal light distributions on the operation site, and
the light fields yield a resultant light field (18) that has a substantially circular shape and that is associated with a light distribution that conforms to a surgical lamp standard (at the moment: DIN EN 60601-2-41:2010) and that has a preset relative illuminance (E_{cx}) conforming to the standard on a predetermined diameter (dₓ) along the tiltable optical axis (28) along which the light field (18) is formed;
a control device (9) for the light sources (26, 27) configured to individually control the light intensity of the fifth light sources (5) and of the at least one sixth light source (27) and to control a tilting angle of the at least one sixth light source (27) such that the preset relative illuminance (E_{cx}) is provided at the predetermined diameter (dₓ) at the chosen distance;
actuating units (33), by means of which the light sources (27, 28) are respectively connected to the control device (9),
a driving device (10) connected to the control device (9) for tilting the at least one sixth light source (7),
a device (24) for detecting a distance between the lamp body (2) and the operating site along the tiltable optical axis (28), and
a means (25) for triggering the change of the individual control of the light intensities of the light sources (26, 27) or the change of the tilting angle of the at least one sixth light source (27),
**characterized in that**
the control device (9) is configured to individually control the light intensities of the individual light sources (26, 27) and to control the tilting angle of the at least one sixth light source (7) such that the predetermined diameter (dₓ) on the operating site where the preset relative illuminance (E_{cx}) of the resultant light field (18) is provided is maintained at a substantially constant value when the distance between the lamp body (2) and the operating site chosen in advance subsequently changes.

7. Surgical lamp (1) according to any of the claims 1 to 6, wherein the predetermined diameter (dₓ) of the resultant light field (18) at a distance that is identical to a maximum working distance is as maximum as large as the smallest predeterminable diameter (dₓ).

8. Surgical lamp (1) according to any of the claims 1 to 6, wherein the predetermined diameter (dₓ) of the resultant light field (18) at a distance that is larger than a maximum working distance is as maximum as large as the smallest predeterminable diameter (dₓ).

9. Surgical lamp (1) according to any of the claims 1 to 8, **characterized in that** the control device (9) is configured such that, when the distance is changed, a central illuminance (E_{c}) of the resultant light field (18) conforming to the standard is maintained at a constant value.

10. Surgical lamp (1) according to any of the claims 1 to 9, **characterized in that** the means (25) for triggering the change of the individual control of the light intensities or the change of the tilting angle is a motion sensor, and the control device (9) is configured such that it evaluates the detected distance between the lamp body (2) and the operating site and accordingly controls the light sources (5, 7) after a completed motion of the lamp body (2) detected by the motion sensor.

11. Surgical lamp (1) according to any of the claims 1 to 10, **characterized in that** the surgical lamp (1) comprises at least one input means (22) connected to the control device (9) for setting the diameter (dₓ) where the preset relative illuminance (E_{cx}) of the resultant light field (18) is provided.

12. Surgical lamp (1) according to claim 11, **characterized in that** the input means (22) is a means for selecting amongst different diameters (dₓ) preset in a selectable manner where the preset relative illuminance (E_{cx}) is provided.

13. Method for operating a surgical lamp (1) according to any of the claims 1 to 12, comprising the following steps:
detecting a change of a distance between the lamp body (2) and the operating site,
adjusting the respective light intensity of the at least one first light source (5) or fifth light source (26) and of the at least third light source (7) or sixth light source (27) and changing the tilting angle of the at least third light source (7) or sixth light source (27) so that the diameter (dₓ) set prior to the change of the distance is maintained at a substantially constant value.

14. Method according to claim 13,
wherein the requested diameter (dₓ) where the preset relative illuminance (E_{cx}) of the resultant light field (18) is provided is set in advance.

15. Method according to any of the claims 13 or 14, wherein the requested central illuminance (E_{c}) of the resultant light field (18) is set in advance.

16. Method according to any of the claims 13 to 15, wherein the adjustment of the diameter (dₓ) where the preset relative illuminance (E_{cx}) of the resultant light field (18) is provided is triggered after the completion of a change of the distance.

17. Method according to any of the claims 14 to 16,
wherein, for preventing an enlargement of the diameter (dₓ) where the preset relative illuminance (E_{cx}) of the resulting light field (18) is provided, the tilting angle of the third light source (7) or of the sixth light source (27) is controlled such that the third light field (17, 17') moves radially in direction towards the optical axis (11) or towards the tiltable optical axis (28), and the first light source (5) or fifth light source (26) generating the first light field (14), and the third light source (7) or the sixth light source (27) generating the third light field (17, 17') are controlled such that the diameter (dₓ) with the relative illuminance (E_{cx}) is maintained substantially unchanged, and
wherein, for preventing a reduction of the diameter (dₓ) where the preset relative illuminance (E_{cx}) of the resulting light field (18) is provided, the tilting angle of the third light source (7) or of the sixth light source (27) is controlled such that the third light field (17, 17') moves radially in a direction away from the optical axis (11) or from the tiltable optical axis (28), and the first light source (5) or the fifth light source (26) and the third light source (7) or the sixth light source (27) are controlled such that the diameter (dₓ) with the relative illuminance (E_{cx}) is maintained substantially unchanged.

18. Method according to claim 17 with a surgical lamp according to claim 9, or claim 9 and any of the claims 10 to 12,
wherein, when preventing the enlargement of the diameter (dₓ) where the preset relative illuminance (E_{cx}) of the resultant light field (18) is provided, the tilting angle of the third light source (7) or of the sixth light source (27) is controlled such that the third light field (17, 17') moves radially in direction towards the optical axis (11) or towards the tiltable optical axis (28) and the first light source (5) or the fifth lights source (26) generating the first light field (14) and the third light source (7) or the sixth lights source (27) generating the third light field (17, 17') are controlled such that the central illuminance (E_{c}) is also maintained at a substantially constant value, and
wherein when preventing of the reduction of the diameter (dₓ) where the preset relative illuminance (E_{cx}) of the resultant light field (18) is provided, the tilting angle of the third light source (7) or of the sixth light source (27) is controlled such that the third light field (17, 17') moves radially in direction away from the optical axis (11) or from the tiltable optical axis (28), and the first light source (5) or the fifth light source (26) and the third light source (7) or the sixth light source (27) are controlled such that also the central illuminance (E_{c}) is maintained at a substantially constant value.

## Revendications

1. Lampe opératoire (1) destinée à permettre d'éclairer un champ opératoire situé à distance sélective le long d'un axe optique (11) comprenant :
un corps de lampe (2) renfermant l'axe optique (11) comportant au moins une première source de lumière (5) et une troisième source de lumière (7),
la première source de lumière (5) étant montée fixe dans le corps de lampe (2) et la troisième source de lumière (7) étant montée basculante dans ce corps de lampe (2),
la première source de lumière (5) forme sur le champ opératoire un premier champ lumineux (14) et la troisième source de lumière (7) forme sur le champ opératoire un troisième champ lumineux (17, 17'), ces champs lumineux ayant respectivement essentiellement le même diamètre (d1) et la même répartition de la lumière, et
les champs lumineux (14, 17, 17') permettent d'obtenir un champ lumineux (18) résultant essentiellement circulaire avec une répartition de la lumière conforme aux normes des lampes opératoires (à l'époque DIN EN 60601-2-41 : 2010) avec une intensité d'éclairage relative conforme aux normes en vigueur préétablies (E_{cx}) pour un diamètre (dₓ) prédéfini sur l'axe optique (11),
un dispositif de commande (9) des sources de lumière (5, 7) qui est adapté pour commander individuellement les intensités lumineuses de la première source de lumière (5) et de la troisième source de lumière (7), et pour commander l'angle de basculement de la troisième source de lumière (7), de façon à obtenir pour la distance choisie, l'intensité d'éclairage relative préréglée (E_{cx}) pour le diamètre prédéfini (dₓ),
des unités de commande (33) par l'intermédiaire desquelles les sources de lumière (5, 7) sont respectivement reliées au dispositif de commande (9),
un dispositif d'entraînement (10) pour faire basculer la troisième source de lumière (7) qui est reliée au dispositif de commande (9),
un dispositif (24) permettant de détecter la distance entre le corps de lampe (2) et le champ opératoire le long de l'axe optique (11), et
des moyens (25) permettant de déclencher une modification de la commande individuelle des intensités lumineuses des sources de lumière (5, 7) ou une modification de l'angle de basculement de la troisième source de lumière (7),
**caractérisée en ce qu'**
en présence d'une modification ultérieure de la distance présélectionnée entre le corps de lampe (2) et le champ opératoire le dispositif de commande (9) est adapté, en étant déclenché par les moyens (25), pour commander individuellement les intensités lumineuses des différentes sources de lumière (5, 7) et pour commander l'angle de basculement de la troisième source de lumière (7) de sorte que le diamètre prédéfini (dₓ) sur le champ opératoire, pour lequel on a l'intensité d'éclairage relative préréglée (E_{cx}) du champ lumineux résultant (10) reste essentiellement non modifié.

2. Lampe opératoire (1) conforme à la revendication 1,
caractérisée en que
la lampe opératoire (1) comprend plusieurs modules, au moins l'une des premières sources de lumière (5) et au moins l'une des troisièmes sources de lumière (7) étant montées dans un module interne, et des troisièmes sources de lumière (7) étant montées dans les autres modules.

3. Lampe opératoire (1) conforme à la revendication 1 ou 2, caractérisée en qu'
une surface de sortie de la lumière (29) du corps de lampe (2) ou du module internes et subdivisée en une zone interne (I) essentiellement circulaire et au moins une zone externe (II) située autour de celle-ci,
la première source de lumière (5) étant située au moins dans la zone interne (I) et la troisième source de lumière (7) étant prévue au moins dans la zone externe (II).

4. Lampe opératoire (1) conforme à l'une des revendications 1 à 3, caractérisée en que
les sources de lumière (5, 7) sont rassemblées en des groupes, au moins un critère de regroupement étant le montage fixe ou mobile des sources de lumière (5, 7), et le dispositif de commande (9) étant adapté de sorte que les sources de lumière (5, 7) puissent être commandées de manière respectivement similaire dans les différents groupes et que les groupes puissent être commandés individuellement.

5. Lampe opératoire (1) conforme à la revendication 4,
**caractérisée en ce que**
le dispositif de commande (9) comprend une zone de mémoire et le dispositif de commande (9) est réalisé de sorte que les intensités lumineuses des sources de lumière (5, 7) dans les différents groupes soient déposées dans la zone de mémoire en tant qu'intensité d'alimentation sous la forme d'un champ de caractéristiques et que l'angle de basculement soit également déposé dans la zone de mémoire, et, en fonction de la distance entre le corps de lampe (2) et le champ opérationnel en paramètres peuvent être appelés sous la forme d'un rapport de mélange par l'appareil de commande (9).

6. Lampe d'opération (1) destinée à permettre d'éclairer un champ opératoire situé à une distance sélective le long d'un axe optique pivotant (28) comprenant :
un corps de lampe (2) équipé de l'axe optique pivotant (28) qui comporte plusieurs cinquièmes sources de lumière (26) et au moins une sixième source de lumière (27), dans laquelle
les cinquièmes sources de lumière (26) sont montées pivotantes dans le corps de lampe (2) et plusieurs des cinquièmes sources de lumière (26) forment un faisceau lumineux résultant avec l'axe optique pivotant (28),
et la sixième source de lumière (27) est montée pivotante dans le corps de lampe (2),
les cinquièmes sources de lumière (26) forment un premier champ lumineux et la sixième source de lumière (27) forme un troisième champ lumineux ayant respectivement essentiellement le même diamètre (d1) et essentiellement la même répartition de la lumière sur le champ opératoire, et
les champs lumineux permettent d'obtenir un champ lumineux résultant (18) essentiellement de forme circulaire avec une répartition de la lumière conforme aux normes des lampes opératoires (à l'époque DIN EN 60601-2-41 : 2010) avec une intensité d'éclairage (E_{cx}) relative conforme aux normes en vigueur préétablies pour un diamètre (dₓ) prédéfini sur l'axe optique pivotant (28) sur lequel est formé le champ lumineux (18),
un dispositif de commande (9) des sources de lumière (26, 27) qui est adapté pour commander individuellement les intensités lumineuses des cinquièmes sources de lumière (8) et de la sixième source de lumière (27), et pour commander l'angle de basculement de la sixième source de lumière (27) de façon à obtenir pour la distance sélectionnée, l'intensité d'éclairage relative préétablie (E_{cx}) pour le diamètre prédéfini (dₓ),
des unités de commande (33) par l'intermédiaire desquelles les sources de lumière (27, 27) sont respectivement reliées au dispositif de commande (9),
un dispositif d'entraînement (10) permettant de faire basculer la sixième source de lumière (7) qui est reliée au dispositif de commande (9),
un dispositif (24) permettant de détecter la distance entre le corps de lampe (2) et le champ opératoire le long de l'axe optique pivotant (28), et des moyens (25) permettant de déclencher une modification de la commande individuelle des intensités lumineuses des sources de lumière (26, 27) ou une modification de l'angle de basculement de la sixième source de lumière (27),
**caractérisée en ce qu'**
en présence d'une modification ultérieure de la distance présélectionnée entre le corps de lampe (2) et le champ opératoire, le dispositif de commande (9) est adapté pour, en étant déclenché par les moyens (25), commander individuellement les intensités lumineuses des différentes sources de lumière (26, 27) et pour commander l'angle de basculement de la sixième source de lumière (7) de sorte que le diamètre prédéfini (dₓ) sur le champ opératoire, pour lequel on a l'intensité d'éclairage relative préétablie (E_{cx}) du champ lumineux (18) résultant reste essentiellement non modifié.

7. Lampe opératoire (1) conforme à l'une des revendications 1 à 6, dans laquelle le diamètre prédéfini (dₓ) du champ lumineux résultant (18) à une distance qui est égale à la distance de travail maximum est au maximum égal au plus petit diamètre (dₓ) pouvant être prédéfini.

8. Lampe opératoire (1) conforme à l'une des revendications 1 à 6, dans laquelle le diamètre (dₓ) prédéfini du champ lumineux résultant (18) à une distance qui est supérieure à la distance de travail maximum est au maximum égal au plus petit diamètre (dₓ) pouvant être prédéfini.

9. Lampe opératoire (1) conforme à l'une des revendications 1 à 8, **caractérisée en ce que**
le dispositif de commande (9) est adapté de sorte que, en présence d'une modification de la distance, l'intensité d'éclairage centrale conforme aux normes en vigueur (E_{c}) du champ lumineux résultant (18) reste inchangée.

10. Lampe d'opération (1) conforme à l'une des revendications 1 à 9, **caractérisée en ce que**
les moyens (25) permettant de déclencher la modification de la commande individuelle des intensités lumineuses ou la modification de l'angle de basculement est un capteur de mouvement, et le dispositif de commande (9) est réalisé de façon à évaluer après achèvement d'un mouvement du corps de lampe (2) détecté par le capteur de mouvement, la distance détectée entre le corps de lampe (2) et le champ opératoire et à commander en conséquence les sources de lumière (5, 7).

11. Lampe opératoire (1) conforme à l'une des revendications 1 à 10, **caractérisée en ce que**
la lampe opération (1) comporte au moins un moyen d'entrée (22) relié au dispositif de commande (9) pour permettre de régler le diamètre (dₓ), pour lequel on a l'intensité d'éclairage relative préétablie (E_{cx}) du champ lumineux résultant (18).

12. Lampe opératoire (1) conforme à la revendication 11,
**caractérisée en ce que**
le moyen d'entrée (22) est un moyen permettant de sélectionner parmi plusieurs diamètres (dₓ) préétablis pouvant être sélectionnés pour lesquels on a l'intensité d'éclairage relative (E_{cx}) préétablie.

13. Procédé de gestion d'une lampe opératoire (1) conforme à l'une des revendications 1 à 12,
comprenant les étapes suivantes consistant à :
détecter une modification de la distance entre le corps de lampe (2) et le champ opératoire,
modifier l'intensité lumineuse respective de la première source de lumière (5) ou de la cinquième source de lumière (26) et de la troisième source de lumière (7) ou de la sixième source de lumière (27), et
modifier l'angle de basculement de la troisième source de lumière (7) ou de la sixième source de lumière (27) de sorte que le diamètre (dₓ) réglé avant la modification de la distance reste essentiellement inchangé.

14. Procédé conforme à la revendication 13, selon lequel le diamètre (dₓ), souhaité pour lequel on a l'intensité d'éclairage relative préréglée (E_{cx}) du champ lumineux résultant (18) est préalablement réglé.

15. Procédé conforme à l'une des revendications 13 ou 14, selon lequel l'intensité d'éclairage centrale (E_{c}) souhaitée du champ lumineux résultant (18) est réglée préalablement.

16. Procédé conforme à l'une des revendications 13 à 15, selon lequel après achèvement d'une modification de la distance, l'adaptation du diamètre (dₓ) pour lequel on a l'intensité d'éclairage relative préréglée (E_{cx}) du champ d'éclairage résultant (18) est déclenchée.

17. Procédé conforme à l'une des revendications 14 et 16, selon lequel pour empêcher une augmentation du diamètre (dₓ) pour lequel on a l'intensité d'éclairage relative préréglée (E_{cx}) du champ lumineux résultant (18), l'angle de basculement de la troisième source de lumière (7) ou de la sixième source de lumière (27) est commandé de sorte que le troisième champ lumineux (17, 17') se déplace radialement en se rapprochant de l'axe optique (11) ou de l'axe optique pivotant (28), et la première source de lumière (5) ou la cinquième source de lumière (26) qui produit le premier champ lumineux (14) et la troisième source de lumière (7) ou la sixième source de lumière (27) qui produit le troisième champ lumineux (17, 17') sont commandées de sorte que le diamètre (dₓ) avec l'intensité d'éclairage relative (E_{cx}) reste essentiellement inchangé, et
pour empêcher une diminution du diamètre (dₓ) pour lequel on a l'intensité d'éclairage (E_{cx}) relative préétablie du champ d'éclairage résultant (18), l'angle de basculement de la troisième source de lumière (7) ou de la sixième source de lumière (27) est commandé de sorte que le troisième champ lumineux (17, 17') se déplace radialement en s'éloignant de l'axe optique (11) ou de l'axe optique pivotant (28), et la première source de lumière (5) ou la cinquième source de lumière (26) et la troisième source de lumière (7) ou la sixième source de lumière (27) sont commandées de sorte que le diamètre (dₓ) avec l'intensité d'éclairage relative (E_{cx}) reste essentiellement inchangé.

18. Procédé conforme à la revendication 17, mis en oeuvre avec une lampe opératoire conforme à la revendication 9 ou à la revendication 9 et à l'une des revendications 10 à 12, selon lequel lors de l'étape consistant à empêcher une augmentation du diamètre (dₓ) pour lequel on a l'intensité d'éclairage (E_{cx}) relative préétablie du champ d'éclairage résultant (18), l'angle de basculement de la troisième source de lumière (7) ou de la sixième source de lumière (27) est commandé de sorte que le troisième champ lumineux (17, 17') se déplace radialement en direction de l'axe optique (11) ou de l'axe optique pivotant (28), la première source de lumière (5) ou la cinquième source de lumière (26) qui produit le premier champ lumineux (14) et la troisième source de lumière (7) ou la sixième source de lumière (27) qui produit le troisième champ lumineux (17, 17') sont commandées de sorte que l'intensité d'éclairage centrale (E_{c}) reste également essentiellement inchangée, et
lors de l'étape consistant à empêcher une diminution du diamètre (dₓ) pour lequel on a l'intensité d'éclairage (E_{cx}) relative préétablie du champ d'éclairage résultant (18) l'angle de basculement de la troisième source de lumière (7) ou de la sixième source de lumière (27) est commandé de sorte que le troisième champ lumineux (17, 17') se déplace radialement en s'éloignant de l'axe optique (11) ou de l'axe optique pivotant (28) et la première source de lumière (5) ou la cinquième source de lumière (26) et la troisième source de lumière (7) ou la sixième source de lumière (27) sont commandées de sorte que l'intensité d'éclairage centrale (E_{c}) reste également essentiellement inchangée.
